# EUROPEAN PATENT APPLICATION

(11) **EP 3 450 563 A1**
(43) Date of publication of application: **06.03.2019**
(21) Application number: 18199895.6
(22) Date of filing: 24.02.2012
(51) Int. Cl.: C12N 15/85, C12N 5/10, A01K 67/027

(54) **GENETICALLY MODIFIED ANIMALS AND METHODS FOR MAKING THE SAME**

(30) Priority: 25.02.2011 US 201161446651 P
(62) Divisional of application: 12749281.7
(71) Applicant: Recombinetics, Inc., Saint Paul, MN 55104 (US); The University Court Of The University of Edinburgh, Edinburgh EH8 9YL (GB)
(72) Inventor: FAHRENKRUG, Scott, C., Minneapolis, MN 55406 (US); CARLSON, Daniel, F., Woodbury, MN 55125 (US); LILLICO, Simon Geoffrey, Midlothian, EH26 8AQ (GB); PALGRAVE, Christopher James, Bristol, BS39 4LH (GB); WHITELAW, Christopher Bruce Alexander, Biggar, ML12 6NS (GB)
(74) Representative: dompatent von Kreisler Selting Werner - Partnerschaft von Patent- und Rechtsanwälten mbB

(57) **Abstract**

Compositions and methods for use of TALENs to make genetically modified livestock are set forth. The methods may include reporters for selecting cells or embryos that have been modified by TALENs for use as progenitor cells to make founder animals.

## Description

### CROSS-REFERENCE TO RELATED APPLICATIONS

This patent claims priority to U.S. Serial No. 61/446,651 filed February 25, 2011, which is hereby incorporated by reference herein for all purposes.

### STATEMENT AS TO FEDERALLY SPONSORED RESEARCH

This invention was made with government support under grant nos. 1R41HL108440-01 "Development of Porcine Genetic Models of Atherosclerosis" awarded by the National Institutes of Health. The United States government has certain rights in the invention.

### TECHNICAL FIELD

The technical field relates to creation of genetically modified animals, for example, livestock animals with functional traits.

### BACKGROUND

Transcription activator-like (TAL) effector sequences can be assembled to bind DNA targets with specificity by assembling sequences of repeat variable-diresidue (RVDs). Fusion proteins of TAL effectors and nucleases (TALENs) can make targeted double-stranded breaks in cellular DNA that can be used to make specific genetic modifications to cells. TALENs have been reported as useful to generate stably modified human embryonic stem cell and induced pluripotent stem cell clones, to generate knockout *C*. *elegans*, and knockout zebrafish.

### SUMMARY

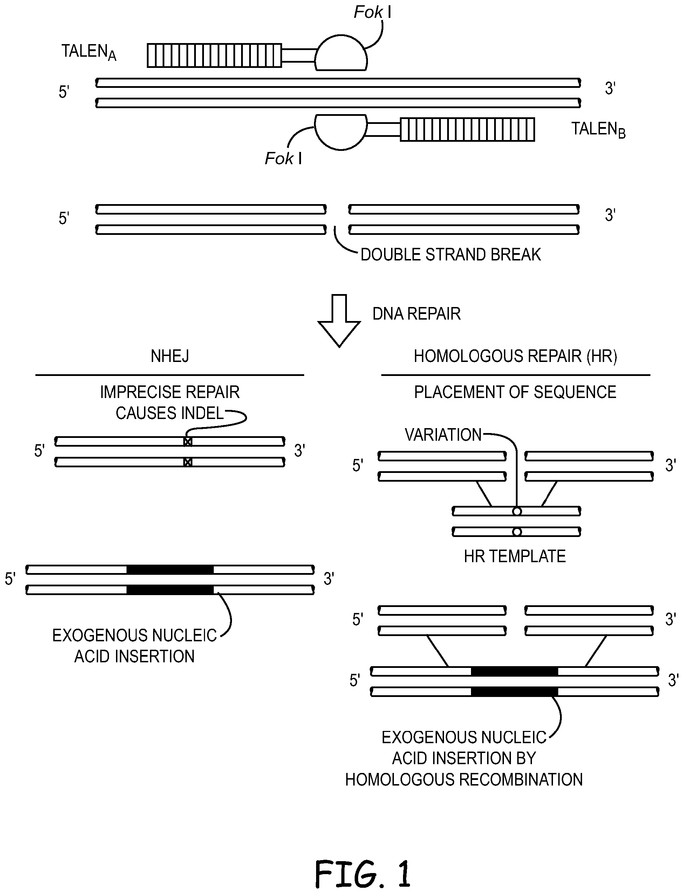

Transcription Activator-Like (TAL) effectors (TALEs) fused with nucleases (TALENs) have been reported for use in genetic modification of cells. These reports have generally focused on plant cells, transformed (immortalized) animal cell lines, or mouse embryonic stem cells. Fig. 1 depicts some of the features of a TALEN-based genetic engineering system.

One embodiment set forth herein is a method of creating a genetic modification comprising exposing a primary cell in an *in vitro* culture or an embryo to a nucleic acid encoding a TALEN, wherein the cell is a livestock cell and the embryo is a livestock embryo. The method may comprise exposing the primary cell to the nucleic acid in th*e in vitro* culture. The nucleic acid encoding a TALEN may comprise an mRNA. The TALEN may be a left TALEN and the method may further comprise a right TALEN that cooperates with the left TALEN to make a double strand cut in a DNA. A second vector may comprise the nucleic acid encoding the TALEN. The method may further comprise exposing the cell or the embryo to a vector encoding a reporter. The method may comprise a vector encoding a selection marker. The reporter may comprise a selection marker. The reporter may require a transposition event performed by a transposase to be expressed. The method may comprise choosing a cell or embryo that expresses the reporter for further processing. The method may comprise wherein the cell is chosen based on elimination of cells from the culture that do not express the reporter. The method may comprise wherein the reporter comprises a fluorescent biomolecule. The method may comprise wherein the embryo is chosen based on expression of the reporter. The method may comprise herein the cell is chosen based on expression of the reporter. The method may comprise wherein the vector comprises a plasmid. The method may comprise wherein the vector comprises a transposon that encodes the reporter, and further comprising a vector that encodes a transposase that recognizes the transposon. The method may comprise herein the transposase is chosen from the group consisting of Sleeping Beauty, Frog Prince, Tol2, Minos, Hsmar, Plaice, and Passport. The method may comprise choosing a cell or embryo that expresses the reporter and using the cell or embryo to create a genetically modified animal, with the genetic modification being at a DNA site specifically bound by the TALEN. The method may comprise breeding the animal and selecting progeny that express the genetic modification and are free of exogenous reporters. The method may comprise wherein the animals are homozygous bi-allelic for the modification, or heterozygous for the modification, or heterozygous bi-allelic for the modification. The method may comprise cloning a genetically modified animal from the cell of the culture wherein the cell comprises a genetic modification at a DNA site specifically bound by the TALEN. The method may comprise herein the cell is used to clone the animal by somatic cell nuclear transfer or chromatin transfer. The method may comprise wherein the genetic modification is chosen from the group consisting of an insertion, a deletion, the insertion of an exogenous nucleic acid fragment, an inversion, a gene conversion to natural allele, gene conversion to a synthetic allele, and a gene conversion to a novel allele. The method may comprise delivering a recombinase to the cell or embryo. The method may comprise wherein the recombinase being is delivered by direct injection or by a vector that encodes the recombinase as a protein, an mRNA, or by a vector that encodes the recombinase. The method may comprise wherein the recombinase combines with a nucleic acid to form a filament, with the nucleic acid providing a template for homology dependent repair. The method may comprise herein the recombinase is chosen from the group consisting of RecA, recA803, uvsX, recA mutants, recA-like recombinases, Cre recombinase, Hin recombinase, RAD51, Tre, FLP, RuvC, DST2, KEM1 XRN1, STPa/DST1, and HPP-1. The method may comprise herein the cell is a zygote, or wherein the embryo is a blastocyst.

Another embodiment set forth herein is a cell or an embryo comprising a nucleic acid fragment encoding a TALEN and a genetic modification at a DNA site that is specifically bound by the TALEN, wherein the cell comprises a primary cell isolated from an animal tissue, and wherein the cell and the embryo are chosen from the group consisting of artiodactyls, swine, bovine, fish, rabbit, and livestock. The primary cell may be, e.g., exposed to the nucleic acid in an *in vitro* culture. The nucleic acid encoding a TALEN may comprise an mRNA. The cell or embryo may comprise a vector encoding a reporter. The cell or embryo may comprise a vector encoding a selection marker. The cell or embryo may comprise wherein the reporter comprises a selection marker. The cell or embryo may comprise a vector encoding a reporter, and a vector that encodes the TALEN. The cell or embryo may comprise a vector encoding a transposase, with the reporter and the TALEN being transposons encoded by their respective vectors. The cell or embryo may be homozygous bi-allelic for the genetic modification. The cell or embryo may comprise an exogenous recombinase.

An embodiment of the invention is a genetically modified livestock animal comprising a genetic modification chosen from the group consisting of an insertion, a deletion, the insertion of an exogenous nucleic acid fragment, an inversion, a gene conversion to natural allele, a gene conversion to a synthetic allele, and a gene conversion to a novel allele, with the animal being free of exogenous reporter genes. The livestock animal may be chosen from the group consisting of artiodactyls, cows, swine, sheep, goats, rabbits, poultry, and fish. The genetic modification may comprise a deletion that comprises deletion of 500 basepairs, or a megabasepair, or an amount of a chromosome ranging from about 10% to about 90%; artisans will immediately appreciate that all the ranges and values within the explicitly stated ranges are contemplated. The genetic modification may comprise a deletion that comprises a megabase of chromosomal DNA. The genetic modification may comprise an inversion that comprises 500 basepairs. The genetic modification may comprise an inversion, with the inversion encoding a genetic trait. The livestock animal may be comprising a bovine line of animals that are not Belgian Blue cattle, wherein the genetic modification comprises a myostatin mutation deletion prevalent in Belgian Blue cattle.

An embodiment is a method of genetically modifying a livestock animal comprising selecting a trait and inverting a region of DNA that encodes the trait in a cell or embryo that is used as a progenitor for a livestock animal. The genetic modification may comprise an in version or a deletion, with the inversion or the deletion comprising 500 basepairs, or a megabasepair, or an amount of a chromosome ranging from about 10% to about 90%; artisans will immediately appreciate that all the ranges and values within the explicitly stated ranges are contemplated.

An embodiment is a method of genetically modifying an animal comprising exposing a progenitor cell or embryo to a first TALEN at a first site and a second TALEN at a second site, with the region between the site being deleted. The first TALEN may comprise a first TALEN pair and the second TALEN may comprise a second TALEN pair. In the case using the progenitor cell, for example, somatic cell nuclear transfer or chromatin transfer from the progenitor cell may be used to create the animal. The method may be a method comprising introducing the TALENs into the progenitor cell and also introducing a vector comprising a reporter, and selecting the progenitor cell or embryo for creation of the animal only if the reporter is expressed.

An embodiment is a method of transfer of an allelic mutation from a first livestock breed to a second livestock breed comprising introducing a TALEN into a cell or embryo of the second livestock breed in a presence of a nucleic acid that encodes the allelic mutation of the first livestock breed wherein the allelic mutation is copied into the cell or the embryo, and creating an animal of the second breed from the cell or the embryo. The copying process may comprise an HDR template. The allelic mutation may comprise, e.g., a myostatin mutation allele present in Belgian Blue cattle. The TALEN may be introduced as a protein or encoded by an mRNA or by a vector. The method may be comprising introduction of a reporter vector independently of the TALEN. The allele may be linked to a trait of the first livestock breed. The method may be further comprising delivering a recombinase to the cell or embryo. The recombinase may be delivered as a protein, an mRNA, or by a vector that encodes the recombinase. The recombinase may be used to combine with the nucleic acid to form a filament.

An embodiment is a genetically modified livestock animal comprising a first breed of livestock animal comprising an allele of a second breed of livestock animal, wherein the first breed of livestock animal is free of meiotic recombination with the second breed of animal. The animal may be, e.g., chosen from the group consisting of swine, cows, sheep, goats, chickens, rabbits, and fish. As an example the first breed is Wagyu cattle and the second breed is Belgian Blue cattle. The allele may be chosen from the group consisting of an insertion, a deletion, a polymorphism, and a single nucleotide polymorphism. The animal may be free of exogenous marker genes. The animal may be comprising a plurality of the alleles. The allele or alleles may be linked to a quantitative trait or qualitative trait of the second breed.

The following patent applications are hereby incorporated herein by reference for all purposes; in case of conflict, the specification is controlling: US 2010/0146655, US 2010/0105140, US 2011/0059160, and US 2011/0197290.

### BRIEF DESCRIPTION OF THE FIGURES

Fig. 1 is an illustration of a TALEN and genetic modifications caused by the same.

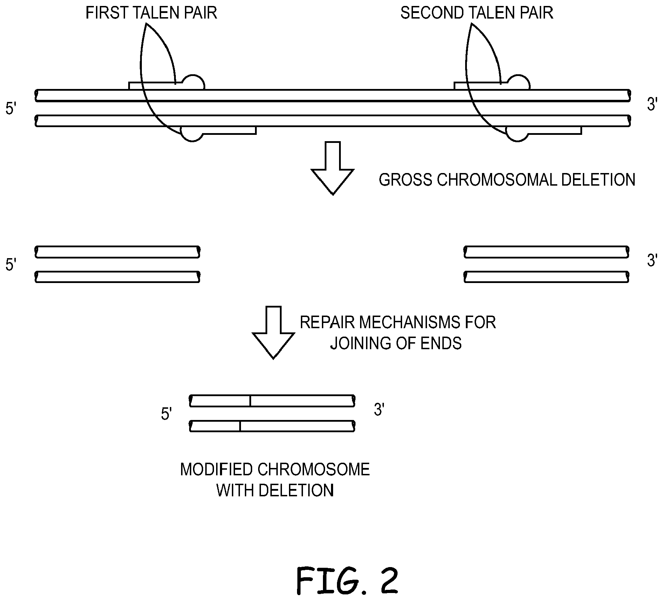

Fig. 2 is an illustration of TALENs operating at a plurality of DNA loci.

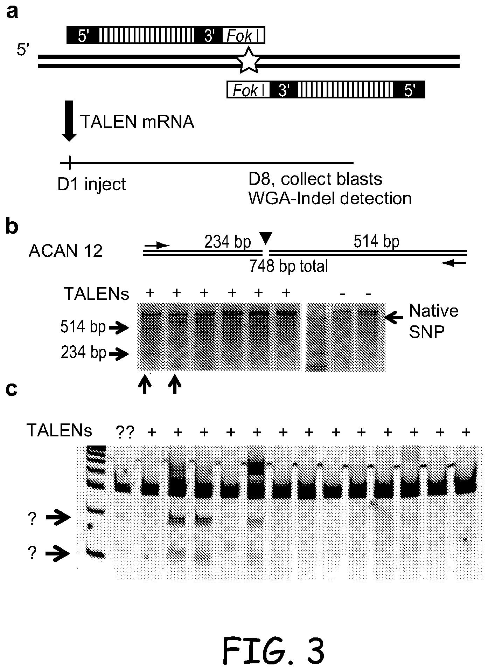

Fig. 3. TALEN activity in bovine embryos. An experimental overview is given in panel (a). TALENs are designed to opposing strands of the DNA target such that the *Fok*I nuclease homodimeric monomers are able to dimerize and cleave DNA between the two monomers. Bovine *in vitro*-produced zygotes are injected with TALEN mRNA on day 1 (D1) and cultured *in vitro* to blastocyst formation. Individual blastocysts (blasts) are collected on day 8, subjected to whole genome amplification (WGA) and analyzed for indels by PCR amplification and Cel-I (SURVEYOR Nuclease, Transgenomics) treatment. Panel b) SURVEYOR Nuclease treatment for analysis of indels in bovine embryos mediated by ACAN12 TALENs. The amplicon length and predicted SURVEYOR cleavage products that are indicative of indels, is shown above. Panel c) SURVEYOR Nuclease treatment for analysis of indels in porcine zygotes mediated by p65 TALENs.

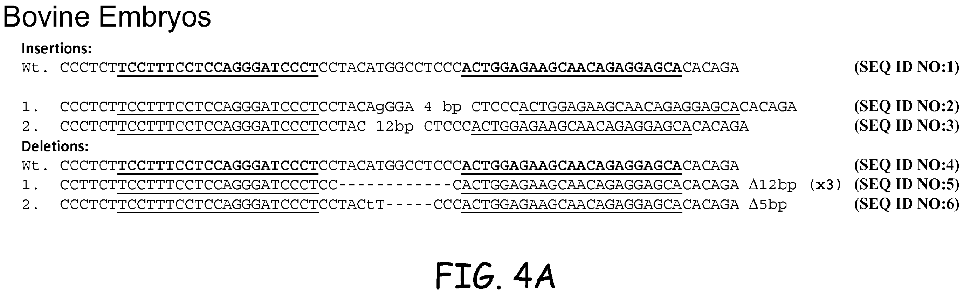

Fig. 4A. Deletions and insertions sequenced from bovine embryos treated with ACAN12 TALENs. The wild-type sequence is shown with TALEN binding sites underlined Both deletion and insertion events were identified.

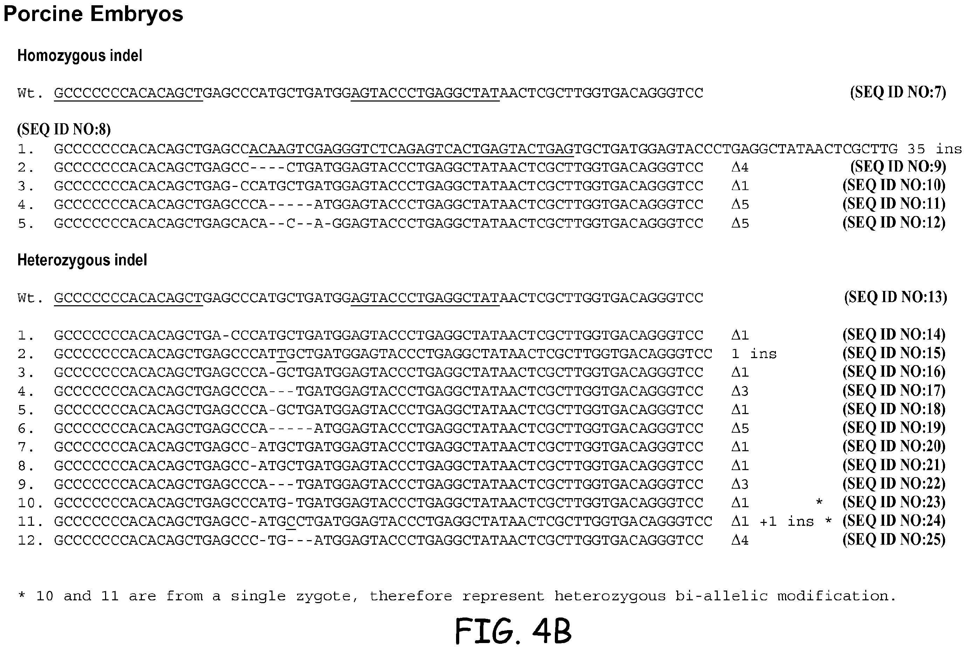

Fig. 4B. Deletions and insertions sequenced from porcine embryos treated with ACAN12 TALENs. The wild-type sequence is shown with TALEN binding sites underlined Both deletion and insertion events were identified.

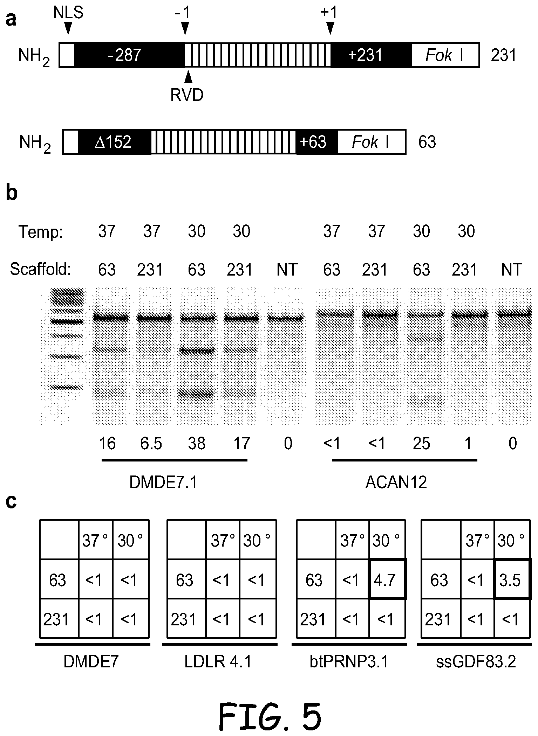

Fig. 5. Comparison of TALEN scaffold for gene editing in livestock fibroblasts Panel a) A diagram of TALEN scaffolds tested in this experiment. Each scaffold (+231, Christian *et. al.* 2010 {Christian, 2010} and +63, Miller *et. al.* 2011 {Miller, 2011}) contains a SV40 nuclear localization signal (NLS) and has a C-terminal fusion of the *Fok*I homodimer domain. Numbering is relative to the DNA binding domain. The amino acid prior to the first repeat variable diresidue repeat (RVD) is labeled "-1" and the amino acid following the last RVD repeat is labeled "+1". Panel b) The SURVEYOR assay was conducted on fibroblasts transfected with either DMDE7.1 or ACAN12 TALEN pairs. Scaffold and temperature treatment is indicated above the gel and percent NHEJ is indicated below. Abbreviations, NT = not treated. Panel c) Activity of four additional TALEN pairs with either the +231 or +63 scaffold.

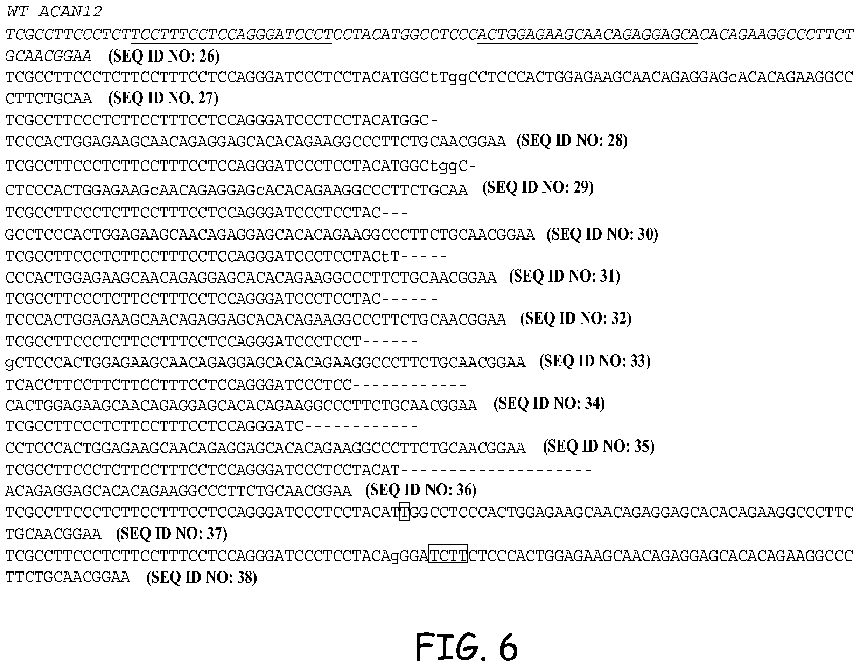

Fig.6. Deletions and insertions sequenced from cells treated with ACAN12 TALENs. The wild-type ACAN12 sequence is displayed in italics and the left and right (complimentary) TALEN-recognition sequences are underlined. Inserted nucleotides are highlighted in grey and mismatch nucleotides are denoted by lower-case text.

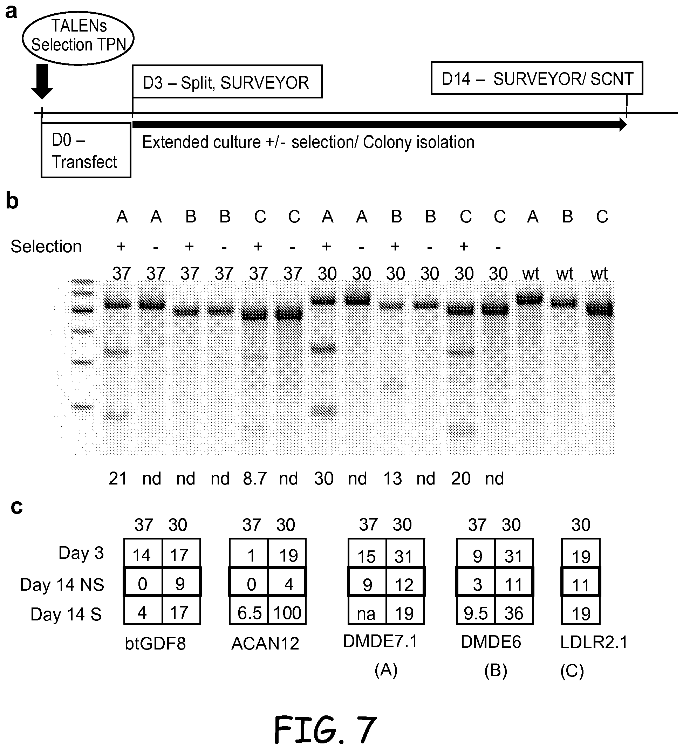

Fig. 7. Transposon co-selection for indel enrichment. An experimental timeline is shown in panel (a). Day zero (D0), cells are transfected with a mixture of plasmids including an expression cassette for each TALEN, a transposon encoding a selection marker, and a transposase-expression cassette. The TALEN plasmid is the major component (4-fold excess by mass) of each transfection. Transfected cells are cultured for 3 days at either 30 or 37 degrees Celsius prior to splitting, collection of a sample for SURVEYOR assay and re-plating for extended culture +/- selection for transposon integration. All cells are cultured at 37 degrees Celsius after day 3. Cells cultured for 14+ days are collected for SURVEYOR assay and cryopreserved for downstream applications, i.e., Single-Cell Nuclear Transfer. Panel b) Fibroblasts were transfected using cationic-lipids. No activity was observed at day 3 (due to low transfection efficiency) so only data for day 14+ populations. Temperature treatment, selection and TALEN id (identified by letters A-C as indicated in panel (c)) are shown above the gel. Panel c) Fibroblasts were transfected by Nucleofection and percent NHEJ was measured at day 3, and in day 14+ non-selected (NS) and selected (S) populations. Temperature treatment is indicated above each matrix. Abbreviations: nd = not detected; wt = wild type amplicon, SURVEYOR treated.

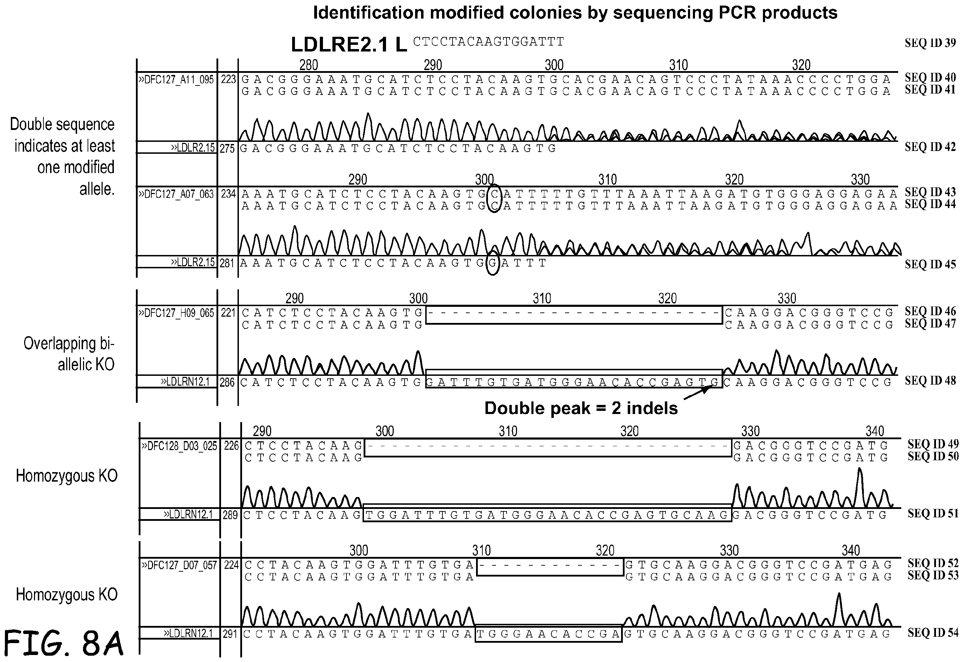

Fig. 8A. Direct PCR sequencing for identification of indels. PCR amplicons from individual fibroblast colonies were purified, sequenced and compared to the wild-type sequence. Mutation of one allele, or, non-overlapping mutations of both alleles will result in double sequence near the TALEN recognition sites (top). Overlapping bi-allelic mutations can be identified where differences between each allele can be identified by double peaks flanking the mutation site. Colonies with homozygous mutations do not display double peaks near the indel site.

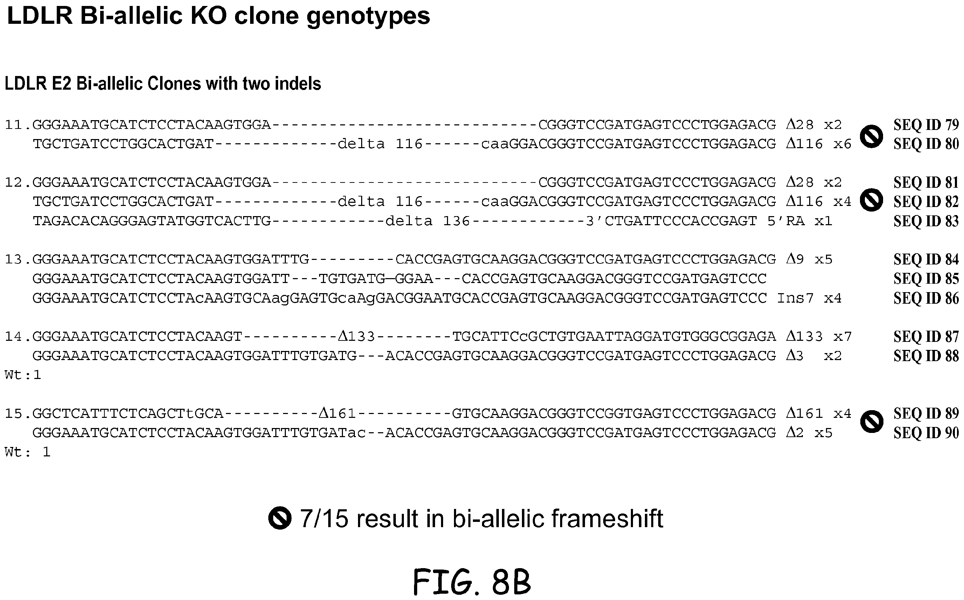

Fig. 8B. Sequence comparisons of wild-type and bi-allelic clones with homozygous indels, as in Fig. 8A.

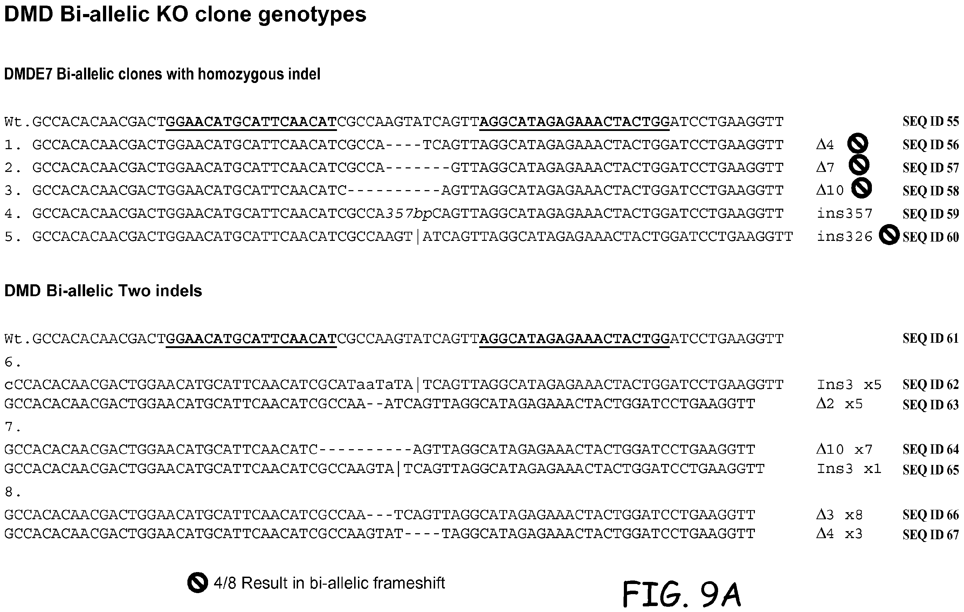

Fig. 9A. DMD Bi-allelic modification alleles. Colonies with either homozygous modification alleles (i.e., both alleles harbor the same mutation) or bi-allelic mutation with different mutations on each allele are displayed. For colonies with two indels, the number of times each allele was sequenced is displayed on the right. In some cases, a third mutation or single wild-type allele was sequenced, indicating that not all colonies are 100% clonal. Frame-shift alleles are indicated and mismatch nucleotides are denoted by lower-case text.

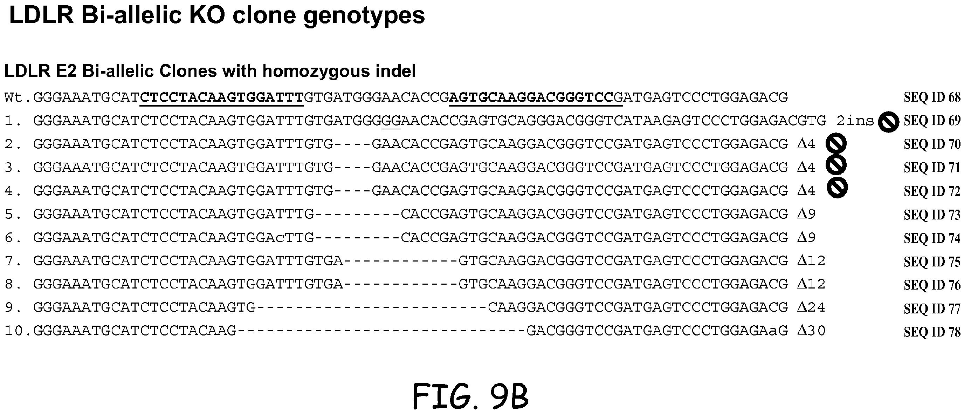

Fig. 9B. LDLR Bi-allelic modification alleles, with notations as in Fig. 9A.

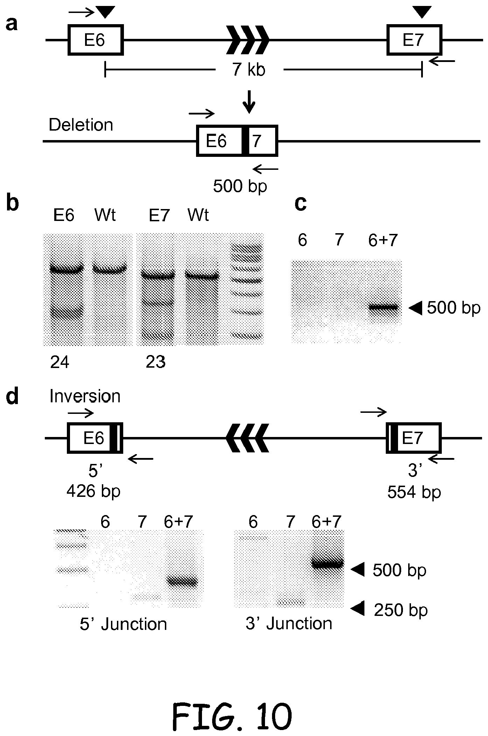

Fig. 10. TALEN-induced deletions and inversions. A schematic of the DMD locus is shown in panel (a). DNA orientation is denoted by black chevrons. TALENs targeted to exons 6 and 7 (black arrowheads) co-transfected into male pig fibroblasts could result in a NHEJ fusion event between exons 6 and 7. This could be identified using primers (black arrows) resulting in ∼500 bp amplicon. Panel b) SURVEYOR assay of cells transfected simultaneously with TALENs targeted to exons 6 and 7 reveal NHEJ indels at both sites. Percent NHEJ is displayed below. Panel c) PCR with primers flanking the presumptive deletion site yield a ∼500 basepair product when both exon-6 and exon-7 TALENs are introduced simultaneously, but not when transfected singly. Panel d) The predicted outcome of an inversion event of the sequence between the TALEN target sites is shown. DNA orientation is denoted by black chevrons. Primers outside the presumptive flanking sites at the 5' and 3' end of the inversion locus are shown (black arrows) along with predicted product size. PCR products were observed at both 5' and 3' junctions only when both exon-6 and exon-7 TALENs are introduced simultaneously.

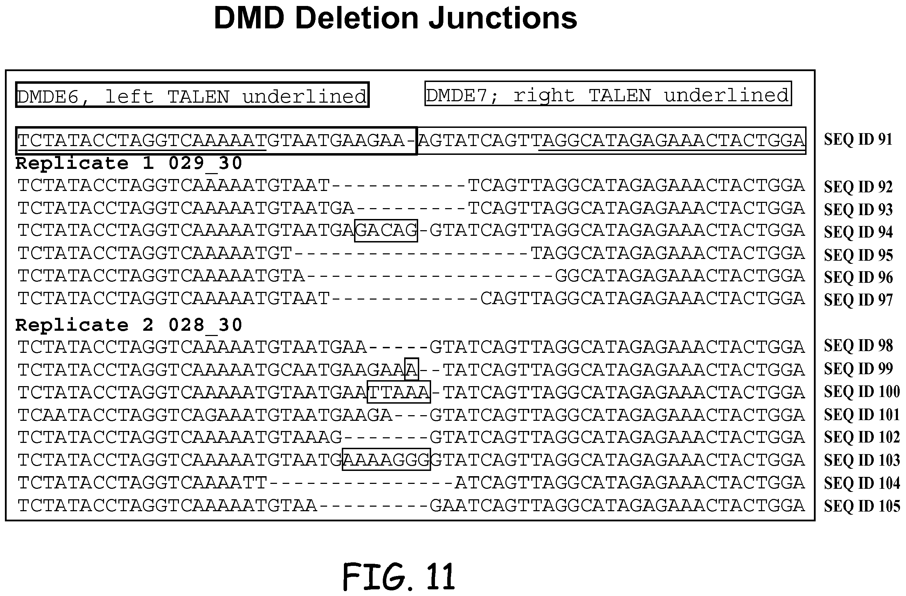

Fig. 11. DMD deletion sequences. DMD deletion junctions from replicate transfections are displayed. Above, exons 6 and 7 sequences are highlighted in green and yellow, respectively, and TALEN-recognition sites are underlined. Inserted nucleotides are highlighted in red.

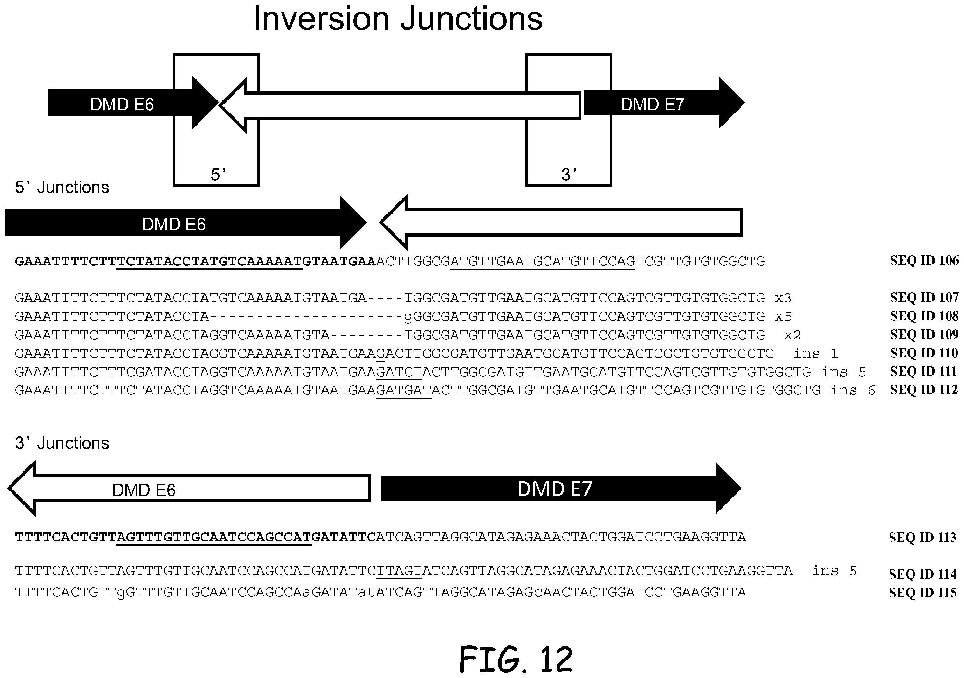

Fig. 12. DMD inversion sequences. A schematic of the DMD inversion allele is shown with the 5' and 3' junctions (boxed) that were analyzed by sequencing. Below, the predicted sequence for each fusion is shown corresponding fusion at the center of each spacer for the TALEN pairs. TALEN-recognition sites are underlined. Sequenced inversion alleles from a transfected population are shown. The number of times each allele was sequenced is indicated at the right and inserted nucleotides are underlined. Mismatched nucleotides are denoted as lower-case text.

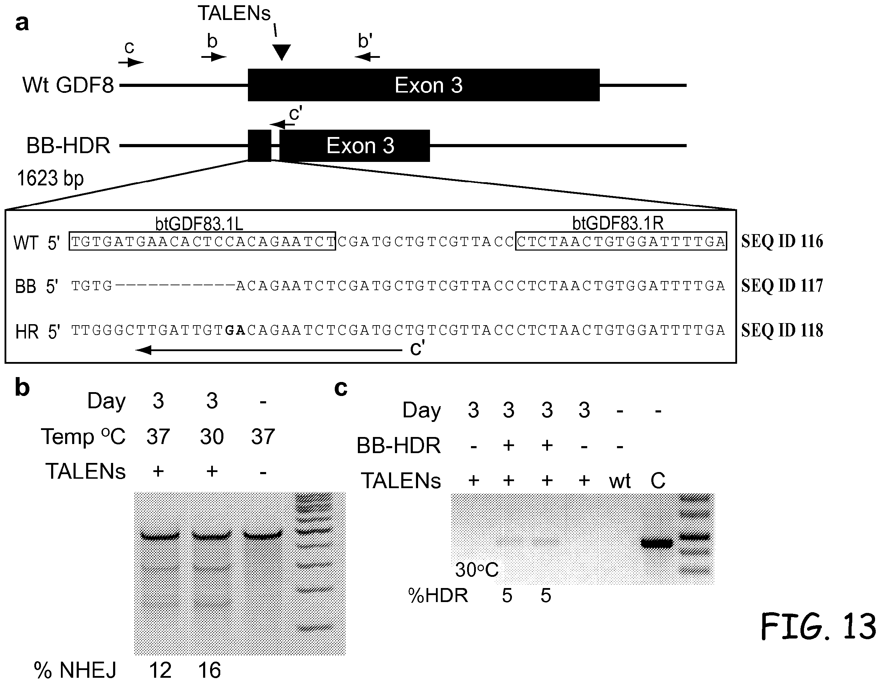

Fig. 13. HDR induction in bovine fibroblasts. Panel a) TALENs (btGDF83.1, arrow) and a dsDNA template (BB-HDR) were designed to introduce an 11-basepair deletion into exon-3 of bovine GDF8 (Belgium Blue mutation) by Double-Strand Break-induced homologous recombination. Half of the binding site for the left TALEN is missing in the BB-HDR template and thus should be resistant to TALEN cleavage. Panel b) SURVEYOR assay demonstrates activity of btGDF83.1 TALENs at both 37 and 30° Celsius. The PCR product used for this assay was generated using primers b and b' (shown in panel a). The BB-HDR template was not included in these replicates since it would confound estimates of btGDF83.1 activity. Panel c) Allele-specific PCR demonstrates that HDR induction is dependent on co-transfection of TALENs and the BB-HDR template. The PCR assay was developed to specifically detect HDR modified GDF8 alleles using primers c and c' (shown panel a). The 3' end of primer c' spans the 11-basepair deletion, and cannot amplify the wild type allele (wt). Five hundred cell equivalents were included in each PCR reaction including the positive control "C". Percent HDR was determined by comparative densitometry between experimental and control reactions.

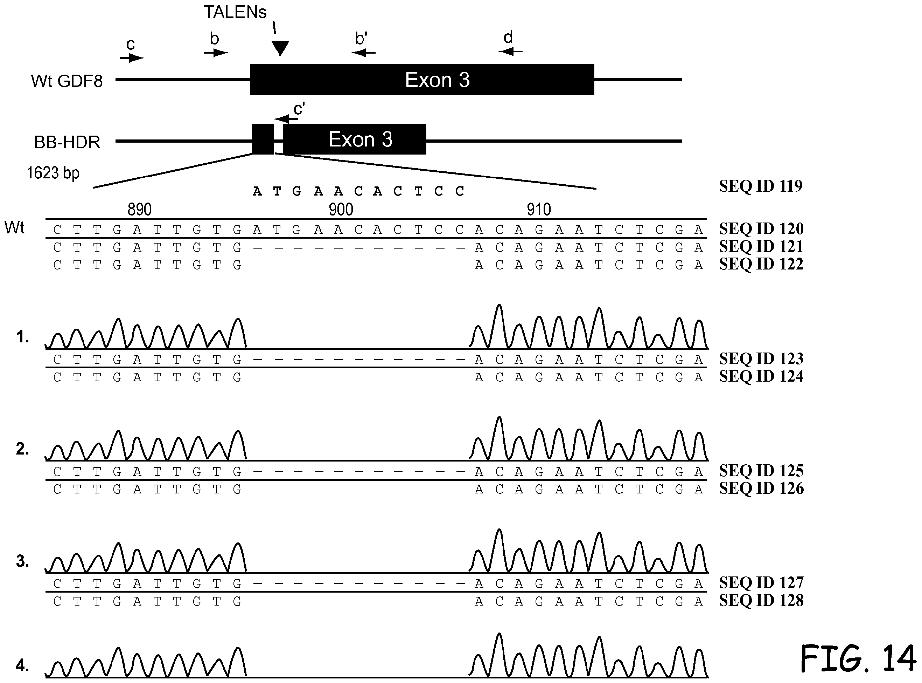

Fig. 14. Confirmation of Belgian Blue introgression by sequencing. The schematics of Wagyu wild-type GDF8 and the Belgian Blue template (BB-HDR) are shown. PCR was conducted using primers located outside of the homology arms (c and d) on five PCR positive colonies followed by cloning and sequencing with primer b'. Comparison to the wild-type sequence reveals the expected 11-basepair deletion characteristic the Belgian Blue allele (heterozygous) in 4 of 5 colonies.

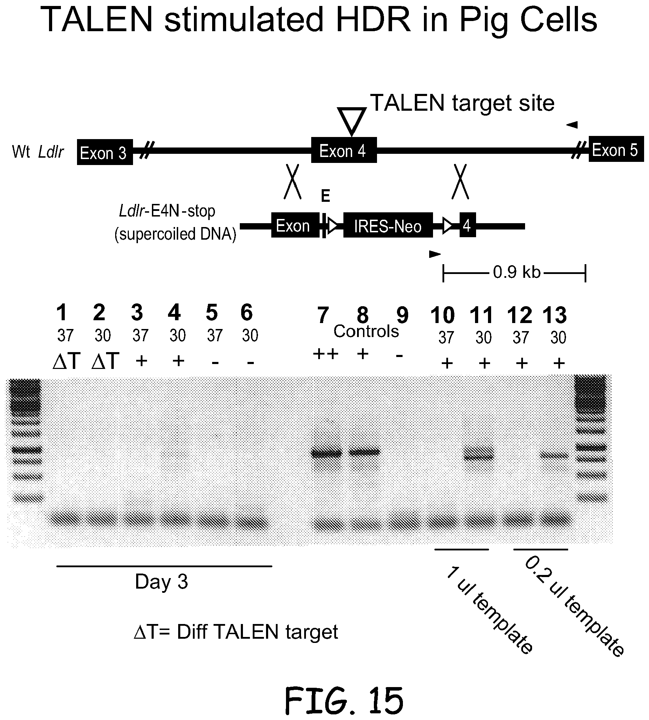

Fig. 15. Schematic and gel for TALEN-mediated HDR. A TALEN pair (LDLR2.1) targeted to the fourth exon of the swine low desity lipoprotein receptor (LDLR) gene was co-transfected with the supercoiled plasmid *Ldlr*-E4N-stop, which contains homology arms corresponding to the swine LDLR gene and a gene-trap enabling expression of Neomycin phosphotransferase upon HDR.

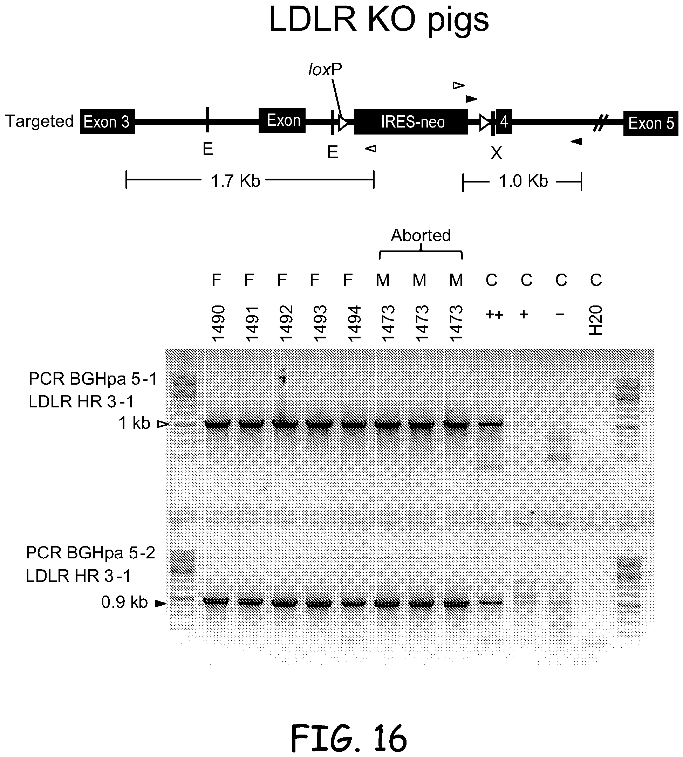

Fig. 16. Schematic and gel for the TALEN-mediated HDR of Fig. 15.

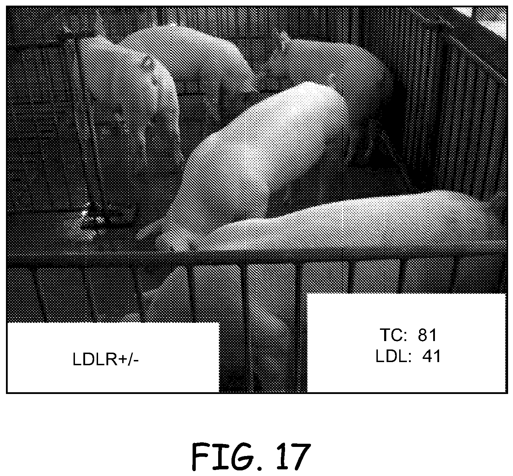

Fig. 17. Transgenic swine created by the processes of Figs. 15 and 16.

### DETAILED DESCRIPTION

The successful use of TALENs to make genetic modifications in higher animals such as swine or cows presents considerable challenges. Compositions and methods of making such animals are set forth herein. Some of these methods involve cloning from primary artiodactyl cells, which also presents considerable challenges. Further, methods for identifying cells or embryos that have been modified with TALENs are presented, as well as processes for enriching the percentage of TALEN-treated cells or embryos. Unexpectedly, it was observed that a genetic modification of one chromosome by a TALEN often caused the complementary locus of the other chromosome to also be modified by cellular machinery.

Further, it was also discovered that TALENs could be used to make gross chromosomal deletions (GCDs) at a plurality of sites. Fig. 2 illustrates this approach, which involves a first TALEN pair directed to a first locus and a second TALEN pair directed to a second locus. It was also surprisingly discovered that inversions of large chromosomal sequences could be created using pairs of TALENs. One use of the inversions is the creation of artiodactyls or other founder animals with fixed genetic traits, or the creation of deletion strains.

### Creation of genetically modified livestock via TALEN technologies; verification of TALEN modification; co-transfectional co-selection for selection of modified cells; elimination of reporter genes from genetically modified livestock

One of the barriers to making TALEN-modified livestock is that the efficiency of making a modification to an animal cell is only a few percent with conventional best practices. Achievement of a deletion or an insertion at an intended site does not necessarily mean success because it may not actually create the intended effect, such as expressing an exogenous protein or stopping expression of an endogenous protein. Even a low efficiency can be useful for the creation of genetically modified lower animals such as fruit flies or mice because they have short and prolific reproductive cycles that provide for the creating, testing, and screening of hundreds of animals to determine if there are a few that have been successfully modified. These levels of efficiency that are conventionally achieved, however, are not suited to livestock artiodactyls that have much longer gestational times and comparatively few progeny per pregnancy.

Another barrier to using TALENs to modify livestock is that TALEN-mediated modification of DNA in primary cells is difficult because the cells are unstable. Indeed, it is shown herein that frequency of TALEN-modified cells decreases significantly over time in the absence of enrichment or selection methods. Without being bound to a particular theory, it is theorized that DNA cleavage at non-intended sites can compromise the stability of the cell by inducing apoptosis or disabling non-target genes. The term primary cell means a cell isolated from a living animal, wherein the cell has undergone between 0 and 2 replications since its isolation from the tissue.

As a result, techniques customarily used to create and test transformed cells for successful genetic modification can not be used in primary cells due to their propensity to senesce. TALEN-modified cells are customarily destroyed to assay their genetic modification, or isolated to grow clonal lines with many identical cells from one parent. However, primary cells are inherently unstable and typically undergo genetic changes, senescence, and/or cell death when attempts are made to genetically modify and clonally expand them. TALEN-modified cells are even less stable, as documented herein for the first time. As a result, it is unreasonable to expect high rates of success when using conventional approaches that involve modifying a primary cell for somatic cell nuclear transfer or other animal cloning technique. As reported herein, however, TALENs have been used to make genetically modified artiodactyl primary cells. These modifications are suited to making founders of genetically modified animal lines by cloning. Also described herein are direct-embryonic injections that were used to modify zygotes, with the modified zygotes being suited to implant into surrogate females for gestation and delivery of founder animal lines.

A typical approach to testing for an actual TALEN-mediated insertion/deletion event is to sequence the modified cell or zygote, which is a destructive process. Thus when a zygote or embryo is modified before implantation to a surrogate, its modification can not be verified with any degree of convenience until the animal is born. It is not conventionally appreciated that an actual production process for making genetically modified animals by cloning will benefit from a process for testing for the presence of a genetic modification. There are inventions presented herein that provide for an indication of genetic modification at the single cell, zygote, or oocyte stage. As shown herein, expression of a reporter gene that is not coupled to TALEN modification is, despite not being part of the reporter gene expression cassette, nonetheless generally predictive of a desired genetic modification. More specifically, the expression of the reporter gene indicates that the nucleic acids were effectively delivered and being expressed in a cell or embryo; a reporter-expressing cell or embryo is more likely to have undergone TALEN-based modification.

Another technique for making modified organisms was the use of a co-transfection, co-selection technique. The cells that express the reporter are selected for, and may be used for making genetically modified animals. The reporter may be chosen to require transposase activity. Without being bound to a specific theory, it is theorized that cells that have undergone transposition have 1) been transfected and 2) been competent for double stranded DNA repair, thus increasing the likelihood of TALEN-based modification in selected clones. This also facilitates enrichment/selection for transposed cells (and by extension TALEN-modified cells). The fact that the transposon is operably but not physically linked to the TALEN modification permits their segregation away from each other by breeding. A benefit of a co-transfection strategy is that the reporter, or reporters, may be placed on a chromosome that is not the same chromosome that is modified by TALENs. This process provides for the creation of founder animals that have no reporter genes. For example, some animals were made by using plasmids carrying reporter genes that were independent of the genetic modification, which was orchestrated separately in the cells. This scheme was based on a theory of operation that cells that incorporate new reporter genes will also incorporate genetic modifications. For instance, data provided herein shows that cells can be transfected with four independent plasmids and the successful incorporation of the gene product of one plasmid is predictive of successful incorporation of the other plasmid gene products and also for the success of TALEN-mediated changes.

TALEN function in livestock embryos was investigated using *in vitro* prepared (IVP) bovine and porcine embryos. Example 1 describes direct injection of TALENs (a left TALEN and a right TALEN) into bovine embryos to produce genetically modified animals with a modification at the site where the TALENs specifically bound. The modifications included homozygous-biallelic and heterozygous-biallelic modifications. TALEN mRNAs were directly injected into the embryos and successful genetic modifications were observed. Example 2 describes additional experiments wherein reporter mRNA was co-injected with TALEN mRNAs. Expression of the reporter was predictive of a successful genetic modification, with about 35% of the embryos expressing the reporter, and about 30% of these animals having a TALEN-based indel. Of the animals with indels, about 35% of them were either homozygous or heterozygous bi-allelic mutants (Fig 4). Direct embryo modification using TALENs was thus shown to be a viable approach to livestock genome modification. Embryos may thus be prepared and implanted into surrogate females for gestation and delivery of animal founder lines using well known processes. Moreover, it is possible to use a reporter to select cells (e.g., primary cells, zygotes, oocytes, blastocysts) for further use in cloning or other processes.

Methods for TALEN-mediated genetic modification of livestock by cloning were also developed. Example 3 describes development of suitable TALENs and TALEN modification of somatic primary cells of swine and cows. The efficiency of successful modification was somewhat low and no reporters for measuring success of the modification were used. Nucleofection is a means for introducing foreign nucleic acids into a cell with high efficiency, but it is expensive, results in high levels of cytotoxicity, and is not available to many researchers. Therefore, a common cationic lipid transfection reagent was used as a vehicle for genetic modification. As shown in Example 4, despite a less than 5% transfection efficiency with cationic lipids, modification levels were significantly enriched by transposon co-selection. Whereas gene modification was below detection in day 3 populations (data not shown) and day 14 populations without transposon-mediated selection, modification levels in selected populations reached 31, 13 and 20 percent for DMD7.1, DMD6 and LDLR2.1 respectively (Fig. 7). Transposon co-selection was then applied to cells transfected by nucleofection where >90% transfection efficiency is routine. Transposon co-selection was effective for maintenance modified cells transfected by Nucleofection, however, with the exception of ACAN12, nucleofection did not significantly enrich for modified cells over day 3 levels (Fig. 7). Thus, transposon co-selection is an effective enrichment method when transfection efficiency is low and an effective maintenance method when transfection efficiency is high. Co-selection processes were also effective when feeder cells were used, as demonstrated in Example 5. An unexpectedly high proportion of bi-allelic modifications (about 17% to about 35% depending on the TALEN-pair) were observed.

An embodiment of the invention is a composition and a method for using TALENs to genetically modify livestock such as artiodactyls. Many of the problems making these animals using conventional processes have been discussed above. The genetic modification may be, for example, chosen from the list consisting of an insertion, a deletion, insertion of an exogenous nucleic acid fragment, an inversion, and gene conversion to a natural or a novel allele. For instance, an undesired mutation in a chromosome or chromosome pair may be replaced with a normal sequence. In general, a target DNA site is identified and a TALEN-pair is created that will specifically bind to the site. The TALEN is delivered to the cell or embryo, e.g., as a protein, mRNA or by a vector that encodes the TALEN. The TALEN cleaves the DNA to make a double-strand break that is then repaired, often resulting in the creation of an indel, or incorporating sequences or polymorphisms contained in an accompanying exogenous nucleic acid that is either inserted or serves as a template for repair of the break with a modified sequence. The term exogenous nucleic acid means a nucleic acid that is added to the cell or embryo, regardless of whether the nucleic acid is the same or distinct from nucleic acid sequences naturally in the cell. The term nucleic acid fragment is broad and includes a chromosome, expression cassette, gene, DNA, RNA, mRNA, or portion thereof. The cell or embryo may be, for instance, chosen from the group consisting of livestock, an artiodactyl, a cow, a swine, a sheep, a goat, a chicken, a rabbit, and a fish. The term livestock means domesticated animals that are raised as commodities for food or biological material. The term artiodactyl means a hoofed mammal of the order *Artiodactyla*, which includes cattle, deer, camels, hippopotamuses, sheep, and goats, that have an even number of toes, usually two or sometimes four, on each foot.

One embodiment is directed to a composition or a method of making a genetically modified livestock and/or artiodactyl comprising introducing a TALEN-pair into livestock and/or an artiodactyl cell or embryo that makes a genetic modification to DNA of the cell or embryo at a site that is specifically bound by the TALEN-pair, and producing the livestock animal/artiodactyl from the cell. Direct injection may be used for the cell or embryo, e.g., into a zygote, blastocyst, or embryo. Alternatively, the TALEN and/or other factors may be introduced into a cell using any of many known techniques for introduction of proteins, RNA, mRNA, DNA, or vectors. Genetically modified animals may be made from the embryos or cells according to known processes, e.g., implantation of the embryo into a gestational host, or various cloning methods. The phrase "a genetic modification to DNA of the cell at a site that is specifically bound by the TALEN", or the like, means that the genetic modification is made at the site cut by the nuclease on the TALEN when the TALEN is specifically bound to its target site. The nuclease does not cut exactly where the TALEN-pair binds, but rather at a defined site between the two binding sites.

Another such embodiment involves a composition or a treatment of a cell that is used for cloning the animal. The cell may be a livestock and/or artiodactyl cell, a cultured cell, a primary cell, a primary somatic cell, a zygote, a germ cell, a primordial germ cell, or a stem cell. For example, an embodiment is a composition or a method of creating a genetic modification comprising exposing a plurality of primary cells in a culture to TALEN proteins or a nucleic acid encoding a TALEN or TALENs. The TALENs may be introduced as proteins or as nucleic acid fragments, e.g., encoded by mRNA or a DNA sequence in a vector.

Genetic modification of animals may also include transfection with a reporter. As discussed above, primary cells were observed to be unstable as a result of cellular modifications mediated by the TALENs and/or TALENs introduction. As a result, success in the modification of primary cells (among other cell types), and/or the creation of new lines of livestock from such cells is not reasonably expected using conventional means. It is theorized, without being bound to a specific theory, that cells that express a gene cassette from a first vector are also likely to be successfully modified by a TALEN delivered independently by mRNA or another vector. Expression of a reporter allows for elimination of cells that do not express the reporter. Alternatively, it allows for moving cells that express the reporter from the culture for use in animals by cloning or other transgenic animal techniques, or into a second culture for further cultivation and/or expansion in number and/or addition of further vectors and/or nucleic acids and/or TALENs and/or other genetic modifications. The term reporter, as used herein, includes reporters and selection makers.

The term selection marker, as used herein, refers to a genetically expressed biomolecule that confers a trait that permits isolation by either positive or negative survival selection criteria. The reporter may be, e.g., a florescent marker, e.g., green fluorescent protein and yellow fluorescent protein. The reporter may be a selection marker, e.g., puromycin, ganciclovir, adenosine deaminase (ADA), aminoglycoside phosphotransferase (neo, G418, APH), dihydrofolate reductase (DHFR), hygromycin-B-phosphtransferase, thymidine kinase (TK), or xanthin-guanine phosphoribosyltransferase (XGPRT).

Embodiments of the invention include introducing a reporter (for instance by use of a vector) and a TALEN (e.g., by an independent vector or mRNA) into a cell or embryo. The cell may be a livestock and/or artiodactyl cell. The TALEN and/or reporter may be directly introduced, e.g., by injection, or other means, e.g., involving cell culture. A cell culture may be made comprising cultured cells (primary cells, zygotes, oocytes, immortalized cells), a first nucleic acid encoding a TALEN, e.g., mRNA or a vector with DNA encoding the TALEN, and an independent vector having a DNA sequence encoding a reporter. The mRNA or first vector do not encode any reporters and the second vector does not encode any TALs and does not encode any TALENs

Vectors for the reporter, selection marker, and/or one or more TALEN may be a plasmid, transposon, transposase, viral, or other vectors,, e.g., as detailed herein. Transposases may be used. One embodiment involving a transposases provides a vector that encodes a transposase. Other vectors encode a transposon that is recognized by the transposase and has a nucleic acid fragment of interest, e.g., a reporter, selection marker, exogenous nucleic acid for insertion or as a template for modification, or one or more TALENs. Accordingly, a cell or embryo may be transfected with a number of vectors between, for example, 1 and about 6; artisans will immediately appreciate that all the ranges and values within the explicitly stated ranges are contemplated, e.g., 2, 3, 4, 5, and 6. More vectors may be used. The reporter may be used to identify cells that are likely to have undergone modification by TALENs. Or a selection marker may be used to enrich the proportion of TALEN-modified cells by destroying cells or embryos that do not express the selection marker.

An embodiment of the invention is a cell or embryo culture exposed to, or injected with, a plurality of vectors. A first vector comprises a TALEN or TALEN-pair; alternatively there are two TALEN vectors that independently provide a left TALEN and a right TALEN. A second vector comprises a reporter. The reporter may provide for non-destructive identification or may be a selection marker. A vector encoding a selection marker may be used as an alternative to the reporter vector, or in addition to the reporter vector. A further vector may encode an exogenous nucleic acid.

A process for making TALEN-modified cells, embryos, or animals comprises assaying a cell or embryo exposed to a TALEN for expression of a reporter and using that cell or embryo in a method or composition for making a genetically modified livestock and/or artiodactyl. For instance, a primary cell may be removed from a cell culture and used for cloning. Or, a primary cell may be removed from culture and placed in a second culture to make a clonal line or for further processes. Or, an embryo or zygote expressing the reporter may be used for either implantation into a surrogate dam or can be used for cloning, while other embryos or zygotes that do not express the reporter not used for cloning. In some embodiments, the reporter is a selection marker that is used to select for cells or embryos that express the marker.

### Gross Chromosomal Deletions and Inversions; Genetically Modified Animals

Experiments were performed with TALENs directed to a plurality of DNA sites. The sites were separated by several thousand base pairs. It was observed that the DNA could be rejoined with the deletion of the entire region between the sites. Embodiments include, for example, sites separated by a distance between 1-5 megabases or between 50% and 80% of a chromosome, or between about 100 and about 1,000,000 basepairs; artisans will immediately appreciate that all the ranges and values within the explicitly stated ranges are contemplated, e.g., from about 1,000 to about 10,000 basepairs or from about 500 to about 500,000 basepairs. Alternatively, exogenous DNA may be added to the cell or embryo for insertion of the exogenous DNA, or template-driven repair of the DNA between the sites. Modification at a plurality of sites may be used to make genetically modified cells, embryos, artiodactyls, and livestock. Example 6 describes the deletion of several thousand basepairs of DNA, with rejoining of the ends verified biochemically.

Unexpectedly, TALEN-cleavage at separated sites also resulted in frequent inversion of the entire region between TALEN targets. As an additional surprise, as detailed in Example 6, these inversions were accomplished with great fidelity. Forty one out of 43 of the tested inversions were positive at both the 5' and 3' junctions. And sequencing of PCR products confirmed both deletion and inversion events with addition or deletion of very few nucleotides at their junctions (Fig. 11, 12). Cells or embryos with these deletions or inversions have many uses as assay tools for genetics.

These cells are also useful for making animals, livestock, and animal models. Large deletions provide for gene inactivation. Also, for instance, a deletion strain may be made of cells, livestock, or animal models. Crossing the deletion strains with an organism bearing a mutation for comparison to a wild-type helps to rapidly and conveniently localize and identify the mutation locus. Deletion strains are well known in these arts and involve sets of organisms made with a series of deletions in a gene. Deletion mapping involves crossing a strain which has a point mutation in a gene with the deletion strains. Wherever recombination occurs between the two strains to produce a wild-type (+) gene, the mutation cannot lie within the region of the deletion. If recombination cannot produce any wild-type genes, then it is reasonable to conclude that the point mutation and deletion are found within the same stretch of DNA. This can be used for example to identify causative mutations, or to identify polymorphisms underlying quantitative trait loci.

Cells, embryos, livestock, artiodactyls, and animal models with inversions are also useful for fixing a genetic trait in progeny of an organism or an animal line or animal breed. Recombinations typically occur between homologous regions of matching chromosomes during meiosis. Inversion of a chromosomal region destroys homology and suppresses meiotic recombination. Methods and compositions described herein may be used to make such organisms or animals. For example, DNA in a somatic bovine or porcine cell may be cut at a plurality of loci by TALENs, and cells with an inversion may be isolated, or cells expressing reporters may be used as likely candidates for successful inversions. The cells may be used to clone animals that harbor chromosomal regions that are incapable of meiotic recombinations. Alternatively, it is expected that inversions will also occur at reasonable frequencies in embryos treated with multiple TALEN-pairs at plurality of sites.

An embodiment of this method is identifying a DNA region encoding a genetic trait and cutting a DNA in a cell or embryo on each side of the encoded trait at sites using a plurality of TALENs. The modified cell or embryo may be used for creating a genetically modified animal. The method may comprise isolating a genetically modified animal that has the inversion.

### Movement of alleles

Some livestock traits are related to alleles such as polymorphisms (large or small), single nucleotide polymorphisms, deletions, insertions, or other variations. For instance, a myostatin allele (an 11-bp deletion) from Belgian Blue cattle is well known to cause a double-muscling phenotype. Example 7 shows, using the Belgian Blue allele, how to precisely transfer specific alleles from one livestock breed to another by homology-dependent repair (HDR). Bovine fibroblasts received the allele and may readily be used to make transgenic cattle. This allele does not interfere with normal development and the methods taught herein place the allele with precision and without disruption of other genes or the incorporation of exogenous genes. As already discussed, results presented herein show that the frequency of allele conversion in livestock fibroblasts is high when sister chromatids are used for an HDR template, therefore allele introgression into one sister chromatid can be anticipated frequently to result in homozygosity.

An embodiment of this invention is a method of transfer of an allele from a first livestock line or breed to a second livestock line or breed, comprising cutting DNA with a pair of TALENs in a cell or embryo of the second livestock line/breed in a presence of a nucleic acid that encodes the allele of the first livestock line/breed. The embryo or cell may be used to create an animal of the second line/breed that has the allele of the first line/breed. The DNA that encodes the allele provides a template for homology-dependent repair. As a template, it has homology to portions of the DNA on each side of the cut and also contains the desired allele. Embodiments of the invention comprise moving a plurality of alleles from one breed to another breed. As set forth elsewhere herein, the TALENs may be delivered a protein or encoded by a nucleic acid, e.g., an mRNA or a vector. A reporter may also be transfected into the cell or embryo and used as a basis for selecting TALEN-modified cells. The reporter may be assayed non-destructively and/or may comprise a selection marker. The term breed means a group of domestic animals or plants with a homogeneous appearance, behavior, and other characteristics that distinguish it from other animals or plants of the same species. The animals that belong to a particular breed are known to artisans that practice in these arts.

A population or species of organisms typically includes multiple alleles at each locus among various individuals. Allelic variation at a locus is measurable as the number of alleles (polymorphisms) present, or the proportion of heterozygotes in the population. For example, at the gene locus for the ABO blood type carbohydrate antigens in humans, classical genetics recognizes three alleles, IA, IB, and IO, that determine compatibility of blood transfusions. An allele is a term that means one of two or more forms of a gene.

In livestock, many alleles are known to be linked to various traits such as production traits, type traits, workability traits, and other functional traits. Artisans are accustomed to monitoring and quantifying these traits, e.g., Visscher et al., Livestock Production Science, 40 (1994) 123-137, US 7,709,206, US 2001/0016315, US 2011/0023140, and US 2005/0153317. Accordingly, the allele that is transferred may be linked to a trait or chosen from a trait in the group consisting of a production trait, a type trait, a workability trait, a fertility trait, a mothering trait, and a disease resistance trait.

The term natural allele in the context of genetic modification means an allele found in nature in the same species of organism that is being modified. The term novel allele means a non-natural allele. A human allele placed into a goat is a novel allele. The term synthetic allele means an allele that is not found in nature. Thus a natural allele is a variation already existing within a species that can be interbred. And a novel allele is one that does not exist within a species that can be interbred.

Moving an allele from one breed to another by conventional breeding processes involves swapping many alleles between the breeds. Recombination during meiosis inevitably exchanges genetic loci between the breeds. In contrast, the TALENs-modified livestock and other animals are free of meiotic recombination events. As a result, a TALEN-modified animal can easily be distinguished from an animal created by sexual reproduction.

### Compositions and kits

The present invention also provides compositions and kits containing, for example, nucleic acid molecules encoding TALENs, TALEN polypeptides, compositions containing such nucleic acid molecules or polypeptides, or TALEN engineered cell lines. Such items can be used, for example, as research tools, or therapeutically.

### Recombinases

Embodiments of the invention include administration of a TALEN or TALENs with a recombinase. A recombinase forms a filament with a nucleic acid fragment and, in effect, searches cellular DNA to find a DNA sequence substantially homologous to the sequence. An embodiment of a TALEN-recombinase embodiment comprises combining a recombinase with a nucleic acid sequence that serves as a template for HDR. The HDR template sequence has substantial homology to a site that is targeted for cutting by the TALEN/TALEN pair. As described herein, the HDR template provides for a change to the native DNA, by placement of an allele, creation of an indel, insertion of exogenous DNA, or with other changes. The TALEN is placed in the cell or embryo by methods described herein as a protein, mRNA, or by use of a vector. The recombinase is combined with the HDR to form a filament and placed into the cell. The recombinase and/or HDR template that combines with the recombinase may be placed in the cell or embryo as a protein, an mRNA, or with a vector that encodes the recombinase. The disclosure of US Pub 2011/0059160 (U.S. Serial No. 12/869,232) is hereby incorporated herein by reference for all purposes; in case of conflict, the specification is controlling. The term recombinase refers to a genetic recombination enzyme that enzymatically catalyzes, in a cell, the joining of relatively short pieces of DNA between two relatively longer DNA strands. Recombinases include Cre recombinase, Hin recombinase, RecA, RAD51, Cre, and FLP. Cre recombinase is a Type I topoisomerase from P1 bacteriophage that catalyzes site-specific recombination of DNA between loxP sites. Hin recombinase is a 21kD protein composed of 198 amino acids that is found in the bacteria Salmonella. Hin belongs to the serine recombinase family of DNA invertases in which it relies on the active site serine to initiate DNA cleavage and recombination. RAD51 is a human gene. The protein encoded by this gene is a member of the RAD51 protein family which assist in repair of DNA double strand breaks. RAD51 family members are homologous to the bacterial RecA and yeast Rad51. Cre recombinase is an experimental enzyme that in lab tests has successfully removed DNA inserted by HIV from infected cells. The enzyme was derived from Cre recombinase through selective mutation for the purposes of identifying HIV markers, which are not bounded by loxP sites and therefore disallow attempts at Cre-Lox recombination. FLP refers to Flippase recombination enzyme (FLP or Flp) derived from the 2µ plasmid of the baker's yeast *Saccharomyces cerevisiae.*

RecA is known for its recombinase activity to catalyze strand exchange during the repair of double-strand breaks by homologous recombination (McGrew and Knight, 2003) Radding, et al., 1981; Seitz et al., 1998). RecA has also been shown to catalyze proteolysis, *e.g*., of the LexA and λ repressor proteins, and to possess DNA-dependent ATPase activity. After a double-strand break occurs from ionizing radiation or some other insult, exonucleases chew back the DNA ends 5' to 3', thereby exposing one strand of the DNA (Cox, 1999; McGrew and Knight, 2003). The single-stranded DNA becomes stabilized by single-strand binding protein (SSB). After binding of SSB, RecA binds the single-stranded (ss) DNA and forms a helical nucleoprotein filament (referred to as a filament or a presynaptic filament). During DNA repair, the homology-searching functions of RecA direct the filament to homologous DNA and catalyze homologous base pairing and strand exchange. This results in the formation of DNA heteroduplex. After strand invasion, DNA polymerase elongates the ssDNA based on the homologous DNA template to repair the DNA break, and crossover structures or Holliday junctions are formed. RecA also shows a motor function that participates in the migration of the crossover structures (Campbell and Davis, 1999).

Recombinase activity comprises a number of different functions. For example, polypeptide sequences having recombinase activity are able to bind in a non-sequence-specific fashion to single-stranded DNA to form a nucleoprotein filament. Such recombinase-bound nucleoprotein filaments are able to interact in a non-sequence-specific manner with a double-stranded DNA molecule, search for sequences in the double-stranded molecule that are homologous to sequences in the filament, and, when such sequences are found, displace one of the strands of the double-stranded molecule to allow base-pairing between sequences in the filament and complementary sequences in one of the strands of the double stranded molecule. Such steps are collectively denoted "synapsis."

RecA and RecA-like proteins (called Rad51 in many eukaryotic species) have been examined for stimulating gene targeting and homologous recombination in a variety of eukaryotic systems. In tobacco cells, expression of bacterial RecA containing a nuclear localization signal (NLS) increases the repair of mitomycin C-induced DNA damage by homologous recombination and somatic intrachromosomal recombination (recombination between homologous chromosomes) from three to ten fold (Reiss et al., 1996). Expression of NLSRecA in tobacco can also stimulate sister chromatid exchange 2.4-fold over wild-type levels (Reiss et al., 2000). In somatic mammalian cells, overexpression of NLSRecA stimulates gene-targeting by homologous recombination 10-fold (Shcherbakova et al., 2000). However, in human cells, overexpression of a human homologue of RecA, hRAD51, only stimulates recombination 2 to 3-fold over wild type levels under the antibiotic selection (Yanez and Porter, 1999). In zebrafish, a mutant form of the enhanced green fluorescent protein (EGFP) was corrected at low frequency by injecting ssDNA-RecA filaments directly (Cui et al., 2003). Rad52, a member of the Rad51 epistasis group, also promotes single-strand annealing and low level gene disruption in zebrafish using mutated oligonucleotides (Takahashi and Dawid, 2005). Taken together, these studies indicate that ectopic expression of RecA or Rad51 results in a modest stimulation of homologous recombination but does not increase levels sufficiently to be useful for gene-targeting.

Thus recombinase activities include, but are not limited to, single-stranded DNA-binding, synapsis, homology searching, duplex invasion by single-stranded DNA, heteroduplex formation, ATP hydrolysis and proteolysis. The prototypical recombinase is the RecA protein from *E. coli.* See, for example, U.S. Patent No. 4,888,274. Prokaryotic RecA-like proteins have also been described in *Salmonella*, *Bacillus* and *Proteus* species. A thermostable RecA protein, from *Thermus aquaticus*, has been described in U.S. Patent No. 5,510,473. A bacteriophage T4 homologue of RecA, the UvsX protein, has been described. RecA mutants, having altered recombinase activities, have been described, for example, in U.S. Patent Nos. 6,774,213; 7,176,007 and 7,294,494. Plant RecA homologues are described in, for example, U.S. Patent Nos. 5,674,992; 6,388,169 and 6,809,183. RecA fragments containing recombinase activity have been described, for example, in U.S. Patent No. 5,731,411. Mutant RecA proteins having enhanced recombinase activity such as, for example, RecA803 have been described. *See*, for example, Madiraju et al. (1988) Proc. Natl. Acad. Sci. USA 85:6592-6596.

A eukaryotic homologue of RecA, also possessing recombinase activity, is the Rad51 protein, first identified in the yeast *Saccharomyces cerevisiae.* See Bishop et al., (1992) Cell 69: 439-56 and Shinohara et al, (1992) Cell: 457-70 Aboussekhra et al., (1992) Mol. Cell. Biol. 72,3224-3234. Basile et al., (1992) Mol. Cell. Biol. 12, 3235-3246.Plant Rad51 sequences are described in U.S. Patent Nos. 6,541,684; 6,720,478; 6,905,857 and 7,034,117. Another yeast protein that is homologous to RecA is the Dmc1 protein. RecA/Rad51 homologues in organisms other than *E. coli* and *S. cerevisiae* have been described. Morita et al. (1993) Proc. Natl. Acad. Sci. USA 90:6577-6580; Shinohara et al. (1993) Nature Genet. 4:239-243; Heyer (1994) Experientia 50:223-233; Maeshima et al. (1995) Gene 160:195-200; U.S. Patent Nos. 6,541,684 and 6,905,857.

Herein, "RecA" or "RecA protein" refers to a family of RecA-like recombination proteins having essentially all or most of the same functions, particularly: (i) the ability to position properly oligonucleotides or polynucleotides on their homologous targets for subsequent extension by DNA polymerases; (ii) the ability topologically to prepare duplex nucleic acid for DNA synthesis; and, (iii) the ability of RecA/oligonucleotide or RecA/polynucleotide complexes efficiently to find and bind to complementary sequences. The best characterized RecA protein is from *E. coli*; in addition to the original allelic form of the protein a number of mutant RecA-like proteins have been identified, for example, RecA803. Further, many organisms have RecA-like strand-transfer proteins including, for example, yeast, *Drosophila,* mammals including humans, and plants. These proteins include, for example, Rec1, Rec2, Rad51, Rad51B, Rad51C, Rad51D, Rad51E, XRCC2 and DMC1. An embodiment of the recombination protein is the RecA protein of *E. coli.* Alternatively, the RecA protein can be the mutant RecA-803 protein of *E. coli*, a RecA protein from another bacterial source or a homologous recombination protein from another organism.

Additional descriptions of proteins having recombinase activity are found, for example, in Fugisawa et al. (1985) Nucl. Acids Res. 13:7473; Hsieh et al. (1986) Cell 44:885; Hsieh et al. (1989) J. Biol. Chem. 264:5089; Fishel et al. (1988) Proc. Natl. Acad. Sci. USA 85:3683; Cassuto et al. (1987) Mol. Gen. Genet. 208:10; Ganea et al. (1987) Mol. Cell Biol. 7:3124; Moore et al. (1990) J. Biol. Chem.: 11108; Keene et al. (1984) Nucl. Acids Res. 12:3057; Kimiec (1984) Cold Spring Harbor Symp. 48:675; Kimeic (1986) Cell 44:545; Kolodner et al. (1987) Proc. Natl. Acad. Sci. USA 84:5560; Sugino et al. (1985) Proc. Natl. Acad, Sci. USA 85: 3683; Halbrook et al. (1989) J. Biol. Chem. 264:21403; Eisen et al. (1988) Proc. Natl. Acad. Sci. USA 85:7481; McCarthy et al. (1988) Proc. Natl. Acad. Sci. USA 85:5854; and Lowenhaupt et al. (1989) J. Biol. Chem. 264:20568, which are incorporated herein by reference. See also Brendel et al. (1997) J. Mol. Evol. 44:528.

Examples of proteins having recombinase activity include recA, recA803, uvsX, and other recA mutants and recA-like recombinases (Roca (1990) Crit. Rev. Biochem. Molec. Biol. 25:415), (Kolodner et al. (1987) Proc. Natl. Acad. Sci. U.S.A. 84:5560; Tishkoff et al. (1991) Molec. Cell. Biol. 11:2593), RuvC (Dunderdale et al. (1991) Nature 354:506), DST2, KEM1 and XRN1 (Dykstra et al. (1991) Molec. Cell. Biol. 11:2583), STPa/DST1 (Clark et al. (1991) Molec. Cell. Biol. 11:2576), HPP-1 (Moore et al. (1991) Proc. Natl. Acad. Sci. U.S.A. 88:9067), other eukaryotic recombinases (Bishop et al. (1992) Cell 69:439; and Shinohara et al. (1992) Cell 69:457); incorporated herein by reference.

*In vitro*-evolved proteins having recombinase activity have been described in U.S. Patent No. 6,686,515. Further publications relating to recombinases include, for example, U.S. Patent Nos. 7,732,585; 7,361,641 and 7,144,734. For a review of recombinases, see Cox (2001) Proc. Natl. Acad. Sci. USA 98:8173-8180.

A nucleoprotein filament, or "filament" may be formed. The term filament, in the context of forming a structure with a recombinase, is a term known to artisans in these fields. The nucleoprotein filament so formed can then be, e.g., contacted with another nucleic acid or introduced into a cell. Methods for forming nucleoprotein filaments, wherein the filaments comprise polypeptide sequences having recombinase activity and a nucleic acid, are well-known in the art. See, e.g., Cui et al. (2003) Marine Biotechnol. 5:174-184 and U.S. Patent Nos. 4,888,274; 5,763,240; 5,948,653 and 7,199,281, the disclosures of which are incorporated by reference for the purposes of disclosing exemplary techniques for binding recombinases to nucleic acids to form nucleoprotein filaments.

In general, a molecule having recombinase activity is contacted with a linear, single-stranded nucleic acid. The linear, single-stranded nucleic acid may be a probe. The methods of preparation of such single stranded nucleic acids are known. The reaction mixture typically contains a magnesium ion. Optionally, the reaction mixture is buffered and optionally also contains ATP, dATP or a nonhydrolyzable ATP analogue, such as, for example, γ-thio-ATP (ATP-γ-S) or γ-thio-GTP (GTP-γ-S). Reaction mixtures can also optionally contain an ATP-generating system. Double-stranded DNA molecules can be denatured (e.g., by heat or alkali) either prior to, or during, filament formation. Optimization of the molar ratio of recombinase to nucleic acid is within the skill of the art. For example, a series of different concentrations of recombinase can be added to a constant amount of nucleic acid, and filament formation assayed by mobility in an agarose or acrylamide gel. Because bound protein retards the electrophoretic mobility of a polynucleotide, filament formation is evidenced by retarded mobility of the nucleic acid. Either maximum degree of retardation, or maximum amount of nucleic acid migrating with a retarded mobility, can be used to indicate optimal recombinase:nucleic acid ratios. Protein-DNA association can also be quantitated by measuring the ability of a polynucleotide to bind to nitrocellulose.

### TALENs

The term TALEN, as used herein, is broad and includes a monomeric TALEN that can cleave double stranded DNA without assistance from another TALEN. The term TALEN is also used to refer to one or both members of a pair of TALENs that are engineered to work together to cleave DNA at the same site. TALENs that work together may be referred to as a left-TALEN and a right-TALEN, which references the handedness of DNA.

Miller et al. (Miller et al. (2011) Nature Biotechnol 29:143) reported making TALENs for site-specific nuclease architecture by linking TAL truncation variants to the catalytic domain of FokI nuclease. The resulting TALENs were shown to induce gene modification in immortalized human cells by means of the two major eukaryotic DNA repair pathways, non-homologous end joining (NHEJ) and homology directed repair. The TALENs can be engineered for specific binding. Specific binding, as that term is commonly used in the biological arts, refers to a molecule that binds to a target with a relatively high affinity compared to non-target tissues, and generally involves a plurality of non-covalent interactions, such as electrostatic interactions, van der Waals interactions, hydrogen bonding, and the like. Specific binding interactions characterize antibody-antigen binding, enzyme-substrate binding, and specifically binding protein-receptor interactions.

The cipher for TALs has been reported (PCT Application WO 2011/072246) wherein each DNA binding repeat is responsible for recognizing one base pair in the target DNA sequence. The residues may be assembled to target a DNA sequence, with: (a) HD for recognition of C/G; (b) NI for recognition of A/T; (c) NG for recognition of T/A; (d) NS for recognition of C/G or A/T or T/A or G/C; (e) NN for 30 recognition of G/C or A/T; (f) IG for recognition of T/A; (g) N for recognition of C/G; (h) HG for recognition of C/G or T/A; (i) H for recognition of T/A; and (j) NK for recognition of G/C. In brief, a target site for binding of a TALEN is determined and a fusion molecule comprising a nuclease and a series of RVDs that recognize the target site is created. Upon binding, the nuclease cleaves the DNA so that cellular repair machinery can operate to make a genetic modification at the cut ends. The term TALEN means a protein comprising a Transcription Activator-like (TAL) effector binding domain and a nuclease domain and includes monomeric TALENs that are functional *per se* as well as others that require dimerization with another monomeric TALEN. The dimerization can result in a homodimeric TALEN when both monomeric TALEN are identical or can result in a heterodimeric TALEN when monomeric TALEN are different.

In some embodiments, a monomeric TALEN can be used. TALEN typically function as dimers across a bipartite recognition site with a spacer, such that two TAL effector domains are each fused to a catalytic domain of the *FokI* restriction enzyme, the DNA-recognition sites for each resulting TALEN are separated by a spacer sequence, and binding of each TALEN monomer to the recognition site allows *FokI* to dimerize and create a double-strand break within the spacer. Monomeric TALENs also can be constructed, however, such that single TAL effectors are fused to a nuclease that does not require dimerization to function. One such nuclease, for example, is a single-chain variant of *FokI* in which the two monomers are expressed as a single polypeptide. Other naturally occurring or engineered monomeric nucleases also can serve this role. The DNA recognition domain used for a monomeric TALEN can be derived from a naturally occurring TAL effector. Alternatively, the DNA recognition domain can be engineered to recognize a specific DNA target. Engineered single-chain TALENs may be easier to construct and deploy, as they require only one engineered DNA recognition domain. A dimeric DNA sequence-specific nuclease can be generated using two different DNA binding domains (e.g., one TAL effector binding domain and one binding domain from another type of molecule). TALENs may function as dimers across a bipartite recognition site with a spacer. This nuclease architecture also can be used for target-specific nucleases generated from, for example, one TALEN monomer and one zinc finger nuclease monomer. In such cases, the DNA recognition sites for the TALEN and zinc finger nuclease monomers can be separated by a spacer of appropriate length. Binding of the two monomers can allow *FokI* to dimerize and create a double-strand break within the spacer sequence. DNA binding domains other than zinc fingers, such as homeodomains, myb repeats or leucine zippers, also can be fused to *FokI* and serve as a partner with a TALEN monomer to create a functional nuclease.

In some embodiments, a TAL effector can be used to target other protein domains (e.g., non-nuclease protein domains) to specific nucleotide sequences. For example, a TAL effector can be linked to a protein domain from, without limitation, a DNA 20 interacting enzyme (e.g., a methylase, a topoisomerase, an integrase, a transposase, or a ligase), a transcription activators or repressor, or a protein that interacts with or modifies other proteins such as histones. Applications of such TAL effector fusions include, for example, creating or modifying epigenetic regulatory elements, making site-specific insertions, deletions, or repairs in DNA, controlling gene expression, and modifying chromatin structure.

The spacer of the target sequence can be selected or varied to modulate TALEN specificity and activity. The flexibility in spacer length indicates that spacer length can be chosen to target particular sequences with high specificity. Further, the variation in activity has been observed for different spacer lengths indicating that spacer length can be chosen to achieve a desired level of TALEN activity.

The term nuclease includes exonucleases and endonucleases. The term endonuclease refers to any wild-type or variant enzyme capable of catalyzing the hydrolysis (cleavage) of bonds between nucleic acids within a DNA or RNA molecule, preferably a DNA molecule. Non-limiting examples of endonucleases include type II restriction endonucleases such as *Fok*I, *Hha*I, *Hin*dlII, *Not*I, *Bbv*Cl, *Eco*RI, *Bgl*II, and *Alw*I. Endonucleases comprise also rare-cutting endonucleases when having typically a polynucleotide recognition site of about 12-45 basepairs (bp) in length, more preferably of 14-45 bp. Rare-cutting endonucleases induce DNA double-strand breaks (DSBs) at a defined locus. Rare-cutting endonucleases can for example be a homing endonuclease, a chimeric Zinc-Finger nuclease (ZFN) resulting from the fusion of engineered zinc-finger domains with the catalytic domain of a restriction enzyme such as FokI or a chemical endonuclease. In chemical endonucleases, a chemical or peptidic cleaver is conjugated either to a polymer of nucleic acids or to another DNA recognizing a specific target sequence, thereby targeting the cleavage activity to a specific sequence. Chemical endonucleases also encompass synthetic nucleases like conjugates of orthophenanthroline, a DNA cleaving molecule, and triplex-forming oligonucleotides (TFOs), known to bind specific DNA sequences. Such chemical endonucleases are comprised in the term "endonuclease" according to the present invention. Examples of such endonuclease include *I-See I, I-Chu L I-Cre I, I-Csm I, PI-See L PI-Tti L PI-Mtu I, I-Ceu I, I-See IL 1- See III, HO, PI-Civ I, PI-Ctr L PI-Aae I, PI-Bsu I, PI-Dha I, PI-Dra L PI-Mav L PI-Meh I, PI-Mfu L PI-Mfl I, PI-Mga L PI-Mgo I, PI-Min L PI-Mka L PI-Mle I, PI-Mma I, PI-30 Msh L PI-Msm I, PI-Mth I, PI-Mtu I, PI-Mxe I, PI-Npu I, PI-Pfu L PI-Rma I, PI-Spb I, PI-Ssp L PI-Fae L PI-Mja I, PI-Pho L PI-Tag L PI-Thy I, PI-Tko I, PI-Tsp I, I-MsoI.*

### Genetically modified animals

Various techniques known in the art can be used to introduce nucleic acid constructs into non-human animals to produce founder lines, in which the nucleic acid construct is integrated into the genome. Such techniques include, without limitation, pronuclear microinjection (U.S. Patent No. 4,873,191), retrovirus mediated gene transfer into germ lines (Van der Putten et al. (1985) Proc. Natl. Acad. Sci. USA 82, 6148-1652), gene targeting into embryonic stem cells (Thompson et al. (1989) Cell 56, 313-321), electroporation of embryos (Lo (1983) Mol. Cell. Biol. 3, 1803-1814), sperm-mediated gene transfer (Lavitrano et al. (2002) Proc. Natl. Acad. Sci. USA 99, 14230-14235; Lavitrano et al. (2006) Reprod. Fert. Develop. 18, 19-23), and *in vitro* transformation of somatic cells, such as cumulus or mammary cells, or adult, fetal, or embryonic stem cells, followed by nuclear transplantation (Wilmut et al. (1997) Nature 385, 810-813; and Wakayama et al. (1998) Nature 394, 369-374). Pronuclear microinjection, sperm mediated gene transfer, and somatic cell nuclear transfer are particularly useful techniques.

Typically, in pronuclear microinjection, a nucleic acid construct is introduced into a fertilized egg; 1 or 2 cell fertilized eggs are used as the pronuclei containing the genetic material from the sperm head and the egg are visible within the protoplasm. Pronuclear staged fertilized eggs can be obtained *in vitro* or *in vivo* (i.e., surgically recovered from the oviduct of donor animals). *In vitro* fertilized eggs can be produced as follows. For example, swine ovaries can be collected at an abattoir, and maintained at 22-28°C during transport. Ovaries can be washed and isolated for follicular aspiration, and follicles ranging from 4-8 mm can be aspirated into 50 mL conical centrifuge tubes using 18 gauge needles and under vacuum. Follicular fluid and aspirated oocytes can be rinsed through pre-filters with commercial TL-HEPES (Minitube, Verona, WI). Oocytes surrounded by a compact cumulus mass can be selected and placed into TCM-199 OOCYTE MATURATION MEDIUM (Minitube, Verona, WI) supplemented with 0.1 mg/mL cysteine, 10 ng/mL epidermal growth factor, 10% porcine follicular fluid, 50 µM 2-mercaptoethanol, 0.5 mg/ml cAMP, 10 IU/mL each of pregnant mare serum gonadotropin (PMSG) and human chorionic gonadotropin (hCG) for approximately 22 hours in humidified air at 38.7°C and 5% CO₂. Subsequently, the oocytes can be moved to fresh TCM-199 maturation medium, which will not contain cAMP, PMSG or hCG and incubated for an additional 22 hours. Matured oocytes can be stripped of their cumulus cells by vortexing in 0.1% hyaluronidase for 1 minute.

For swine, mature oocytes can be fertilized in 500 µl Minitube PORCPRO IVF MEDIUM SYSTEM (Minitube, Verona, WI) in Minitube 5-well fertilization dishes. In preparation for *in vitro* fertilization (IVF), freshly-collected or frozen boar semen can be washed and resuspended in PORCPRO IVF Medium to 4 x 10⁵ sperm. Sperm concentrations can be analyzed by computer assisted semen analysis (SPERMVISION, Minitube, Verona, WI). Final *in vitro* insemination can be performed in a 10µl volume at a final concentration of approximately 40 motile sperm/oocyte, depending on boar. Incubate all fertilizing oocytes at 38.7°C in 5.0% CO₂ atmosphere for 6 hours. Six hours post-insemination, presumptive zygotes can be washed twice in NCSU-23 and moved to 0.5 mL of the same medium. This system can produce 20-30% blastocysts routinely across most boars with a 10-30% polyspermic insemination rate.

Linearized nucleic acid constructs can be injected into one of the pronuclei. Then the injected eggs can be transferred to a recipient female (e.g., into the oviducts of a recipient female) and allowed to develop in the recipient female to produce the transgenic animals. In particular, *in vitro* fertilized embryos can be centrifuged at 15,000 X g for 5 minutes to sediment lipids allowing visualization of the pronucleus. The embryos can be injected with using an Eppendorf FEMTOJET injector and can be cultured until blastocyst formation. Rates of embryo cleavage and blastocyst formation and quality can be recorded.

Embryos can be surgically transferred into uteri of asynchronous recipients. Typically, 100-200 (e.g., 150-200) embryos can be deposited into the ampulla-isthmus junction of the oviduct using a 5.5-inch TOMCAT® catheter. After surgery, real-time ultrasound examination of pregnancy can be performed.

In somatic cell nuclear transfer, a transgenic artiodactyl cell (e.g., a transgenic pig cell or bovine cell) such as an embryonic blastomere, fetal fibroblast, adult ear fibroblast, or granulosa cell that includes a nucleic acid construct described above, can be introduced into an enucleated oocyte to establish a combined cell. Oocytes can be enucleated by partial zona dissection near the polar body and then pressing out cytoplasm at the dissection area. Typically, an injection pipette with a sharp beveled tip is used to inject the transgenic cell into an enucleated oocyte arrested at meiosis 2. In some conventions, oocytes arrested at meiosis-2 are termed "eggs." After producing a porcine or bovine embryo (e.g., by fusing and activating the oocyte), the embryo is transferred to the oviducts of a recipient female, about 20 to 24 hours after activation. See, for example, Cibelli et al. (1998) Science 280, 1256-1258 and U.S. Patent No. 6,548,741. For pigs, recipient females can be checked for pregnancy approximately 20-21 days after transfer of the embryos.

Standard breeding techniques can be used to create animals that are homozygous for the target nucleic acid from the initial heterozygous founder animals. Homozygosity may not be required, however. Transgenic pigs described herein can be bred with other pigs of interest.

In some embodiments, a nucleic acid of interest and a selectable marker can be provided on separate transposons and provided to either embryos or cells in unequal amount, where the amount of transposon containing the selectable marker far exceeds (5-10 fold excess) the transposon containing the nucleic acid of interest. Transgenic cells or animals expressing the nucleic acid of interest can be isolated based on presence and expression of the selectable marker. Because the transposons will integrate into the genome in a precise and unlinked way (independent transposition events), the nucleic acid of interest and the selectable marker are not genetically linked and can easily be separated by genetic segregation through standard breeding. Thus, transgenic animals can be produced that are not constrained to retain selectable markers in subsequent generations, an issue of some concern from a public safety perspective.

Once transgenic animal have been generated, expression of a target nucleic acid can be assessed using standard techniques. Initial screening can be accomplished by Southern blot analysis to determine whether or not integration of the construct has taken place. For a description of Southern analysis, see sections 9.37-9.52 of Sambrook et al., 1989, Molecular Cloning, A Laboratory Manual, second edition, Cold Spring Harbor Press, Plainview; NY. Polymerase chain reaction (PCR) techniques also can be used in the initial screening. PCR refers to a procedure or technique in which target nucleic acids are amplified. Generally, sequence information from the ends of the region of interest or beyond is employed to design oligonucleotide primers that are identical or similar in sequence to opposite strands of the template to be amplified. PCR can be used to amplify specific sequences from DNA as well as RNA, including sequences from total genomic DNA or total cellular RNA. Primers typically are 14 to 40 nucleotides in length, but can range from 10 nucleotides to hundreds of nucleotides in length. PCR is described in, for example PCR Primer: A Laboratory Manual, ed. Dieffenbach and Dveksler, Cold Spring Harbor Laboratory Press, 1995. Nucleic acids also can be amplified by ligase chain reaction, strand displacement amplification, self-sustained sequence replication, or nucleic acid sequence-based amplified. See, for example, Lewis (1992) Genetic Engineering News 12,1; Guatelli et al. (1990) Proc. Natl. Acad. Sci. USA 87:1874; and Weiss (1991) Science 254:1292. At the blastocyst stage, embryos can be individually processed for analysis by PCR, Southern hybridization and splinkerette PCR (see, e.g., Dupuy et al. Proc Natl Acad Sci USA (2002) 99:4495).

Expression of a nucleic acid sequence encoding a polypeptide in the tissues of transgenic pigs can be assessed using techniques that include, for example, Northern blot analysis of tissue samples obtained from the animal, *in situ* hybridization analysis, Western analysis, immunoassays such as enzyme-linked immunosorbent assays, and reverse-transcriptase PCR (RT-PCR).

### Vectors and Nucleic acids

A variety of nucleic acids may be introduced into the artiodactyl or other cells, for knockout purposes, or to obtain expression of a gene for other purposes. Nucleic acid constructs that can be used to produce transgenic animals include a target nucleic acid sequence. As used herein, the term nucleic acid includes DNA, RNA, and nucleic acid analogs, and nucleic acids that are double-stranded or single-stranded (i.e., a sense or an antisense single strand). Nucleic acid analogs can be modified at the base moiety, sugar moiety, or phosphate backbone to improve, for example, stability, hybridization, or solubility of the nucleic acid. Modifications at the base moiety include deoxyuridine for deoxythymidine, and 5-methyl-2'-deoxycytidine and 5-bromo-2'-doxycytidine for deoxycytidine. Modifications of the sugar moiety include modification of the 2' hydroxyl of the ribose sugar to form 2'-O-methyl or 2'-O-allyl sugars. The deoxyribose phosphate backbone can be modified to produce morpholino nucleic acids, in which each base moiety is linked to a six membered, morpholino ring, or peptide nucleic acids, in which the deoxyphosphate backbone is replaced by a pseudopeptide backbone and the four bases are retained. See, Summerton and Weller (1997) Antisense Nucleic Acid Drug Dev. 7(3):187; and Hyrup et al. (1996) Bioorgan. Med. Chem. 4:5. In addition, the deoxyphosphate backbone can be replaced with, for example, a phosphorothioate or phosphorodithioate backbone, a phosphoroamidite, or an alkyl phosphotriester backbone.

The target nucleic acid sequence can be operably linked to a regulatory region such as a promoter. Regulatory regions can be porcine regulatory regions or can be from other species. As used herein, operably linked refers to positioning of a regulatory region relative to a nucleic acid sequence in such a way as to permit or facilitate transcription of the target nucleic acid.

Any type of promoter can be operably linked to a target nucleic acid sequence. Examples of promoters include, without limitation, tissue-specific promoters, constitutive promoters, and promoters responsive or unresponsive to a particular stimulus. Suitable tissue specific promoters can result in preferential expression of a nucleic acid transcript in beta cells and include, for example, the human insulin promoter. Other tissue specific promoters can result in preferential expression in, for example, hepatocytes or heart tissue and can include the albumin or alpha-myosin heavy chain promoters, respectively. In other embodiments, a promoter that facilitates the expression of a nucleic acid molecule without significant tissue- or temporal-specificity can be used (i.e., a constitutive promoter). For example, a beta-actin promoter such as the chicken beta-actin gene promoter, ubiquitin promoter, miniCAGs promoter, glyceraldehyde-3-phosphate dehydrogenase (GAPDH) promoter, or 3-phosphoglycerate kinase (PGK) promoter can be used, as well as viral promoters such as the herpes simplex virus thymidine kinase (HSV-TK) promoter, the SV40 promoter, or a cytomegalovirus (CMV) promoter. In some embodiments, a fusion of the chicken beta actin gene promoter and the CMV enhancer is used as a promoter. See, for example, Xu et al. (2001) Hum. Gene Ther. 12:563; and Kiwaki et al. (1996) Hum. Gene Ther. 7:821.

An example of an inducible promoter is the tetracycline (tet)-on promoter system, which can be used to regulate transcription of the nucleic acid. In this system, a mutated Tet repressor (TetR) is fused to the activation domain of herpes simplex virus VP16 transactivator protein to create a tetracycline-controlled transcriptional activator (tTA), which is regulated by tet or doxycycline (dox). In the absence of antibiotic, transcription is minimal, while in the presence of tet or dox, transcription is induced. Alternative inducible systems include the ecdysone or rapamycin systems. Ecdysone is an insect molting hormone whose production is controlled by a heterodimer of the ecdysone receptor and the product of the *ultraspiracle* gene (USP). Expression is induced by treatment with ecdysone or an analog of ecdysone such as muristerone A. The agent that is administered to the animal to trigger the inducible system is referred to as an induction agent.

Additional regulatory regions that may be useful in nucleic acid constructs, include, but are not limited to, polyadenylation sequences, translation control sequences (e.g., an internal ribosome entry segment, IRES), enhancers, inducible elements, or introns. Such regulatory regions may not be necessary, although they may increase expression by affecting transcription, stability of the mRNA, translational efficiency, or the like. Such regulatory regions can be included in a nucleic acid construct as desired to obtain optimal expression of the nucleic acids in the cell(s). Sufficient expression, however, can sometimes be obtained without such additional elements.

A nucleic acid construct may be used that encodes signal peptides or selectable markers. Signal peptides can be used such that an encoded polypeptide is directed to a particular cellular location (e.g., the cell surface). Non-limiting examples of selectable markers include puromycin, ganciclovir, adenosine deaminase (ADA), aminoglycoside phosphotransferase (neo, G418, APH), dihydrofolate reductase (DHFR), hygromycin-B-phosphtransferase, thymidine kinase (TK), and xanthin-guanine phosphoribosyltransferase (XGPRT). Such markers are useful for selecting stable transformants in culture. Other selectable markers include fluorescent polypeptides, such as green fluorescent protein or yellow fluorescent protein.

In some embodiments, a sequence encoding a selectable marker can be flanked by recognition sequences for a recombinase such as, e.g., Cre or Flp. For example, the selectable marker can be flanked by *loxP* recognition sites (34-bp recognition sites recognized by the Cre recombinase) or FRT recognition sites such that the selectable marker can be excised from the construct. See, Orban, et al., Proc. Natl. Acad. Sci. (1992) 89:6861, for a review of Cre/lox technology, and Brand and Dymecki, Dev. Cell (2004) 6:7. A transposon containing a Cre- or Flp-activatable transgene interrupted by a selectable marker gene also can be used to obtain transgenic animals with conditional expression of a transgene. For example, a promoter driving expression of the marker/transgene can be either ubiquitous or tissue-specific, which would result in the ubiquitous or tissue-specific expression of the marker in F0 animals (e.g., pigs). Tissue specific activation of the transgene can be accomplished, for example, by crossing a pig that ubiquitously expresses a marker-interrupted transgene to a pig expressing Cre or Flp in a tissue-specific manner, or by crossing a pig that expresses a marker-interrupted transgene in a tissue-specific manner to a pig that ubiquitously expresses Cre or Flp recombinase. Controlled expression of the transgene or controlled excision of the marker allows expression of the transgene.

In some embodiments, the target nucleic acid encodes a polypeptide. A nucleic acid sequence encoding a polypeptide can include a tag sequence that encodes a "tag" designed to facilitate subsequent manipulation of the encoded polypeptide (e.g., to facilitate localization or detection). Tag sequences can be inserted in the nucleic acid sequence encoding the polypeptide such that the encoded tag is located at either the carboxyl or amino terminus of the polypeptide. Non-limiting examples of encoded tags include glutathione S-transferase (GST) and FLAG™ tag (Kodak, New Haven, CT).

In other embodiments, the target nucleic acid sequence induces RNA interference against a target nucleic acid such that expression of the target nucleic acid is reduced. For example the target nucleic acid sequence can induce RNA interference against a nucleic acid encoding a cystic fibrosis transmembrane conductance regulatory (CFTR) polypeptide. For example, double-stranded small interfering RNA (siRNA) or small hairpin RNA (shRNA) homologous to a CFTR DNA can be used to reduce expression of that DNA. Constructs for siRNA can be produced as described, for example, in Fire et al. (1998) Nature 391:806; Romano and Masino (1992) Mol. Microbiol. 6:3343; Cogoni et al. (1996) EMBO J. 15:3153; Cogoni and Masino (1999) Nature 399:166; Misquitta and Paterson (1999) Proc. Natl. Acad. Sci. USA 96:1451; and Kennerdell and Carthew (1998) Cell 95:1017. Constructs for shRNA can be produced as described by McIntyre and Fanning (2006) BMC Biotechnology 6:1. In general, shRNAs are transcribed as a single-stranded RNA molecule containing complementary regions, which can anneal and form short hairpins.

Nucleic acid constructs can be methylated using an *Sss*I CpG methylase (New England Biolabs, Ipswich, MA). In general, the nucleic acid construct can be incubated with S-adenosylmethionine and *Sss*I CpG-methylase in buffer at 37°C. Hypermethylation can be confirmed by incubating the construct with one unit of *Hin*P1I endonuclease for 1 hour at 37°C and assaying by agarose gel electrophoresis.

Nucleic acid constructs can be introduced into embryonic, fetal, or adult artiodactyl cells of any type, including, for example, germ cells such as an oocyte or an egg, a progenitor cell, an adult or embryonic stem cell, a primordial germ cell, a kidney cell such as a PK-15 cell, an islet cell, a beta cell, a liver cell, or a fibroblast such as a dermal fibroblast, using a variety of techniques. Non-limiting examples of techniques include the use of transposon systems, recombinant viruses that can infect cells, or liposomes or other non-viral methods such as electroporation, microinjection, or calcium phosphate precipitation, that are capable of delivering nucleic acids to cells.

In transposon systems, the transcriptional unit of a nucleic acid construct, i.e., the regulatory region operably linked to a target nucleic acid sequence, is flanked by an inverted repeat of a transposon. Several transposon systems, including, for example, *Sleeping Beauty* (see, U.S. Patent No. 6,613,752 and U.S. Publication No. 2005/0003542); Frog Prince (Miskey et al. (2003) Nucleic Acids Res. 31:6873); Tol2 (Kawakami (2007) Genome Biology 8(Suppl.1):S7; *Minos* (Pavlopoulos et al. (2007) Genome Biology 8(Suppl.1):S2); *Hsmar1* (Miskey et al. (2007)) Mol Cell Biol. 27:4589); and Passport have been developed to introduce nucleic acids into cells, including mice, human, and pig cells. The *Sleeping Beauty* transposon is particularly useful. A transposase can be delivered as a protein, encoded on the same nucleic acid construct as the target nucleic acid, can be introduced on a separate nucleic acid construct, or provided as an mRNA (e.g., an *in vitro*-transcribed and capped mRNA).

Insulator elements also can be included in a nucleic acid construct to maintain expression of the target nucleic acid and to inhibit the unwanted transcription of host genes. See, for example, U.S. Publication No. 2004/0203158. Typically, an insulator element flanks each side of the transcriptional unit and is internal to the inverted repeat of the transposon. Non-limiting examples of insulator elements include the matrix attachment region-(MAR) type insulator elements and border-type insulator elements. See, for example, U.S. Patent Nos. 6,395,549; 5,731,178; 6,100,448; and 5,610,053, and U.S. Publication No. 2004/0203158.

Nucleic acids can be incorporated into vectors. A vector is a broad term that includes any specific DNA segment that is designed to move from a carrier into a target DNA. A vector may be referred to as an expression vector, or a vector system, which is a set of components needed to bring about DNA insertion into a genome or other targeted DNA sequence such as an episome, plasmid, or even virus/phage DNA segment. Vector systems such as viral vectors (e.g., retroviruses, adeno-associated virus and integrating phage viruses), and non-viral vectors (e.g., transposons) used for gene delivery in animals have two basic components: 1) a vector comprised of DNA (or RNA that is reverse transcribed into a cDNA) and 2) a transposase, recombinase, or other integrase enzyme that recognizes both the vector and a DNA target sequence and inserts the vector into the target DNA sequence. Vectors most often contain one or more expression cassettes that comprise one or more expression control sequences, wherein an expression control sequence is a DNA sequence that controls and regulates the transcription and/or translation of another DNA sequence or mRNA, respectively.

Many different types of vectors are known. For example, plasmids and viral vectors, e.g., retroviral vectors, are known. Mammalian expression plasmids typically have an origin of replication, a suitable promoter and optional enhancer, and also any necessary ribosome binding sites, a polyadenylation site, splice donor and acceptor sites, transcriptional termination sequences, and 5' flanking non-transcribed sequences. Examples of vectors include: plasmids (which may also be a *carrier* of another type of vector), adenovirus, adeno-associated virus (AAV), lentivirus (e.g., modified HIV-1, SIV or FIV), retrovirus (e.g., ASV, ALV or MoMLV), and transposons (e.g., *Sleeping Beauty*, P-elements, *Tol-2*, *Frog Prince*, *piggyBac*).

As used herein, the term nucleic acid refers to both RNA and DNA, including, for example, cDNA, genomic DNA, synthetic (e.g., chemically synthesized) DNA, as well as naturally occurring and chemically modified nucleic acids, e.g., synthetic bases or alternative backbones. A nucleic acid molecule can be double-stranded or single-stranded (i.e., a sense or an antisense single strand). The term transgenic is used broadly herein and refers to a genetically modified organism or genetically engineered organism whose genetic material has been altered using genetic engineering techniques. A knockout artiodactyl is thus transgenic regardless of whether or not exogenous genes or nucleic acids are expressed in the animal or its progeny.
**Example 1** Genetically modified artiodactyl livestock (bovine) produced by direct injection of TALENs:
   Three TALEN pairs were designed and assembled as described in Cermak et. al. (2011) Nuc. Acids Res. 39:e82 (Fig. 3) and mRNA was injected into the cytoplasm of bovine embryos at about 19 hours post fertilization. Injected embryos were cultured *in vitro* and collected at the blastocysts stage (Fig. 3). Individual blastocyst genomic DNA was amplified by whole genome amplification (WGA) (Carlson et al. (2011) Trangenic Res. 20:29) and screened for indels using the SURVEYOR nuclease assay (Fig. 3, 4A, and 4B). Cleavage products indicative of TALEN activity were observed in 10% of injected embryos (e.g., Fig. 3, 4A, and 4B). Mutations in the predicted region were confirmed in 2 blastocysts injected with either ACAN11 or ACAN12 TALEN pairs (Fig.3). A significant decrease in the developmental competence of TALEN-injected embryos was not observed. A second round of injections was then performed using the ACAN12 TALEN pair at mRNA dosages ranging from 10-100 ng/µl. Comparison of the blastocyst formation rate between rounds 1 (33%) and 2 (5%) (10 ng/µl conditions) revealed poor embryo quality (Table S2). Despite the poor quality of the embryos, 12 putative mutants (27% of injected) were identified using the SURVEYOR assay. The genotypes of each SURVEYOR positive embryo were analyzed with 14 sequencing reads from cloned PCR products. Sequencing revealed mosaicism in gene modification. Indels were identified in 4 SURVEYOR positive embryos and of these, indel positive sequence reads accounted for 7-29% of the total reads for each embryo. These results demonstrated that TALENs functioned in artiodactyl embryos. Gene modification of embryos with Zing Finger Nucleases (ZFNs) and TALENs has been reported for model organisms by direct injection of ZFN or TALEN mRNAs encoding a nuclease pair Geurts et al. (2009) Science 325:433; Carbery et al., (2010) Genetics 186:451; Mashimo et al. (2010) PLoS One 5:e8870; Tesson et al. (2011) Nature Biotechnol. 29:695.
**Example 2** Genetically modified artiodactyl livestock (Swine) produced by direct injection of TALENs.
   A single set of injections was also conducted in porcine IVP embryos using TALENs targeted to the porcine RELA gene (p65) for which a tolerance allele for African Swine Fever has been proposed (Palgrave et al. (2011) J. Virol. 85:6008). Two groups reported enhancement in TALEN activity using truncated variants of the native TALE scaffolds (Mussolino et al. (2011) Nucleic Acids Res. 39:9283; Miller et al. (2011) Nature Biotechnol. 29:143 {Mussolino, 2011 #151;Miller, 2011 #42}. Therefore, in contrast to experiments above which used the +231 scaffold *(Bam*HI fragment, Christian et. al. (2010) Genetics 186:757), the p65 TALEN scaffold was chosen to truncate to mimic the +63 version described in Miller et. al. 2011 (*op cit*) (also see Fig. 5). Zygotes were injected with a mixture of mRNA including 10ng/µl each for the left and right TALENs along with 5ng/µl EGFP mRNA as an indicator of successful injection. Seventy five EGFP positive zygotes were identified (of 214 injected, 35%) and subjected to WGA indel analysis. PCR amplification was successful from 56 of the EGFP positives embryos, and 16 of these (29%) revealed indels by SURVEYOR assay (Fig. 3) or sequence analysis. Mosaicism appears to be reduced in the porcine zygotes compared to cattle, in fact, one third of the mutants (6/16) were either homozygous or heterozygous bi-allelic mutants (Fig 4). These results demonstrated that TALENs functioned in artiodactyl embryos. Processes for the creation of animal founder lines based on modified embryos are well known.
**Example 3** Genetically modified artiodactyl livestock produced by genetic modification of bovine and swine somatic cells.
   Several additional TALEN pairs were assembled for targets in pigs and cattle chosen based on either biomedical or agricultural relevance. Binding domains of six TALEN pairs were placed in the context of two previously described TALEN scaffolds (+231, Christian *et. al.* 2010 (*op cit*) and +63, Miller *et. al.* 2011 (*op cit*)) (Fig. 5). Each TALEN pair was transfected into primary livestock fibroblasts, and genome modification was measured at day 3 by the SURVEYOR assay (Guschin, *et al.* (2010) *Methods Mol. Biol.* 649:247. The most active TALEN pairs, DMDE7.1 and ACAN12, displayed cleavage of 38% and 25% of chromosomes, respectively, and Sanger sequencing revealed an assortment of indels characteristic of NHEJ mediated DNA repair (Fig. 5 and Fig. 6). The TALENs scaffold had a significant effect on activity in fibroblasts. In total, 4 of 6 loci targeted with the +63 isoform cleaved at 3.5% or greater while only the DMDE7.1 TALEN pair cleaved above 1% in the +231 scaffold (Fig. 5). As noted in previous studies Doyon et al. (2010) Nature Methods 8:74; Miller, (2011) *op. cit*.), a 72hour incubation at 30°C after transfection also had a positive effect on activity, and was required for activity of 3 TALEN pairs. The success rate for generating active TALEN pairs has been high. Data collected up to the time of filing shows that 23 of 36 (64%) TALEN pairs were detectably active (> 1.0% NHEJ) at 15 genes scattered across the pig and cow genome, on autosomes and both the X and Y chromosomes (Table S3). Three quarters of the active pairs cleaved with high efficiency (19-40%) with an average modification level of 25%. Clonal processes for the creation of animal founder lines based on modified fibroblasts are well known.
**Example 4** Extended culture and indel enrichment by transposon co-transfection.
   TALEN pairs were transfected into fibroblasts and cultured cells for 14+ days with or without transposon co-selection prior to measurement of modification levels. The results are summarized in Fig. 7. At day zero (D0), cells are transfected with a mixture of plasmids including an expression cassette for each TALEN (two plasmids), a transposon encoding a selection marker (a third plasmid, encoding puromycin, and a transposase-expression cassette (fourth plasmid). The TALEN plasmids are the main component (4-fold excess by mass) of each transfection. Transfected cells are cultured for 3 days at either 30 or 37 degrees Celsius prior to splitting, collection of a sample for SURVEYOR assay and re-plating for extended culture +/- selection for transposon integration. All cells are cultured at 37 degrees Celsius after day 3. Cells cultured for 14+ days are collected for SURVEYOR assay and cryopreserved for downstream applications, i.e., SCNT. For comparison, other fibroblasts were transfected by Nucleofection and percent NHEJ was measured at day 3, and in day 14+ non-selected (NS) and selected (S) populations. For comparison, fibroblasts were also transfected using cationic-lipids.
**Example 5** Isolation of mono- and bi-allelic KO clones.
   Transgenic primary fibroblasts can be effectively expanded and isolated as colonies when plated with non-transgenic fibroblasts (feeder-cells) at standard densities (> 150 cells/cm²) and subjected to drug selection using the transposon co-selection technique applied above (Carlson et al. (2011) Transgenic Res. 20:1125). To evaluate this approach, puromycin resistant colonies for cells treated with six TALEN pairs were isolated and their genotypes evaluated by SURVEYOR assay or direct sequencing of PCR products spanning the target site (Fig. 8A and 8B). In general, the proportion of indel positive clones was similar to predictions made based on day 3 modification levels. Bi-alleic knockout clones were identified for 5 of 6 TALEN pairs, occurring in up to 35 percent of indel positive cells (Table 1). In the majority of examples (15 of 23), indels were homozygous (same indel on each allele) rather than unique indels on each allele suggesting that sister chromatid-templated repair is common (Fig. 9). Notably, among modified clones, the frequency of bi-alleic modification (17-35 %) for the majority of TALEN pairs exceed predictions based on day 3 modification (10-15.6 %) if chromosome cleavages are treated as independent events. Previous studies with ZFN (Kim et al. (2009) Genome Res. 19:1279; Lee et al. (2010) Genome Res. 20:81; Perez et al. (2008) Nature Biotechnol. 26:808) are in support of these results. These previous studies suggested that bi-allelic modification occurs in nearly one-third of modified cells independent of the nuclease activity level.
**Example 6** Chromosomal deletions and inversions with TALENs.
   It was hypothesized that simultaneous delivery of two TALEN pairs targeting the same chromosome could induce large chromosomal deletions or inversions. The TALEN pairs, DMDE6 and DMDE7.1 were tested because of their high activity and the fact that a high percentage of Duchene's Muscular Dystrophy is caused by gross deletions (Blake, 2002) such that a porcine model would match to the human condition. The results are summarized in Fig. 10. Day 3 gene modification levels were high for each TALEN pair (24% for DMDE6 and 23% DMDE7.1), albeit slightly lower that when either TALEN pair was transfected individually (Fig. 10). To determine if the sequence between the two TALEN pairs had been deleted, PCR was attempted with primers spanning the TALEN target sites. If the 6.5 kb sequence had been removed, a band of ∼500 bp was expected, whereas the wild type band of 7 kb would not be amplified with the PCR conditions chosen. A band near 500 bp was observed in replicates where both TALEN pairs were introduced, but was absent when either TALEN pair was introduced alone (Fig. 10).
   Next, the cell population was assayed for inversion events by PCR across presumptive new 5' and 3' junctions. Products were observed at the expected size for both the 5' and 3' junctions of the presumptive inversion only when both TALEN pairs were introduced (Fig. 10). To validate further that the inversions, colonies were generated using the transposon co-selection strategy and screened for deletion and inversion events. Both deletion and inversion events were recovered with high frequency (10.3% and 4.1% respectively; n > 1000) (Table S4). Deletion and inversion events occurred with remarkable fidelity. Forty one out of 43 of the inversion positive colonies were positive at both the 5' and 3' junctions. Finally, sequencing of PCR products confirmed both deletion and inversion events with addition or deletion of very few nucleotides at their junctions (Fig. 11, 12).
**Example 7** TALEN-induced homologous recombination eliminates need for linked selection markers.
   A mutant myostatin allele (an 11 bp deletion) from Belgian Blue cattle was placed into the genome of wild-type Wagyu cattle (Grobet et al. (1997) Nature Genet. 17:71) (Fig. 13). When transfected alone, the btGDF8.1 TALEN pair cleaved up to 16% of chromosomes at the target locus (Fig. 13). TALENs (btGDF83.1) and a dsDNA template (BB-HDR) were designed to introduce an 11-bp deletion in exon-3 of bovine GDF8 (Belgium Blue mutation) by DSB induced homologous recombination. Half of the binding site for the left TALEN was missing in the BB-HDR template, to make it resistant to TALEN cleavage. A SURVEYOR assay demonstrated activity of btGDF83.1 TALENs at both 37 and 30° Celsius. The PCR product used for this assay was generated using primers b and b' (shown panel a of Fig. 13). The BB-HDR template was not included in these replicates since it would confound estimates of btGDF83.1 activity. Allele specific PCR demonstrated that HDR induction was dependent on co-transfection of TALENs and the BB-HDR template. The PCR assay was developed to specifically detect HDR modified GDF8 alleles using primers c and c' (shown panel a of Fig. 13). The 3' end of primer c' spanned the 11-bp deletion, and cannot amplify the wild type allele "wt". Five hundred cell equivalents were included in each PCR reaction including the positive control "C". Percent HDR was determined by comparative densitometry between experimental and control reactions. Co-transfection with a supercoiled DNA template harboring a 1623bp DNA fragment from Belgian Blue cattle resulted in a gene conversion frequency (HDR) of up to 5% as suggested by semi-quantitative PCR at day 3, without selection for the desired event (Fig. 13). These results demonstrated that TALENs can be used to effectively place exogenous nucleic acid sequences in livestock, including alleles - and without markers. To assess the frequency of placement in individual colonies, the transposon co-selection strategy was implemented to isolate and expand individual colonies for DNA sequencing. Gene conversion using template from Belgian Blue cattle was detected in 5 colonies out of 366 examined by PCR. Amplification with primers outside the Belgian Blue HDR template and sequencing confirmed the presence of the expected 11 bp deletion in 4 of the colonies (Fig. 14).
**Example 8** TALEN mediated DNA cleavage as a target for HDR in livestock cells.
   A TALEN pair (LDLR2.1) targeted to the fourth exon of the swine low density lipoprotein receptor (LDLR) gene was co-transfected with the supercoiled plasmid *Ldlr*-E4N-stop, which contains homology arms corresponding to the swine LDLR gene and a gene-trap enabling expression of Neomycin phosphotransferase upon HDR (Figure 15). After 3 days of culture PCR analysis revealed that, even without antibiotic selection, a band corresponding to an HDR event can be detected at the targeted locus (lane 4) at 30°C. Selection of populations of cultured cells for 14 days with geneticin (G418) resulted in significant enrichment of HDR cells (lanes 11 & 13). Cloning of such modified cells by somatic cell nuclear transfer resulted in the birth of 8 pigs (5 gilts, 3 boars), each containing the TALEN-mediated HDR event (Figure 16) as evidenced by flanking PCR on both sides of the homology arms. The gilts are shown in Fig. 17, and these pigs display the intended phenotype of postprandial dyslipidemia compared with normal pigs as shown in the table below:

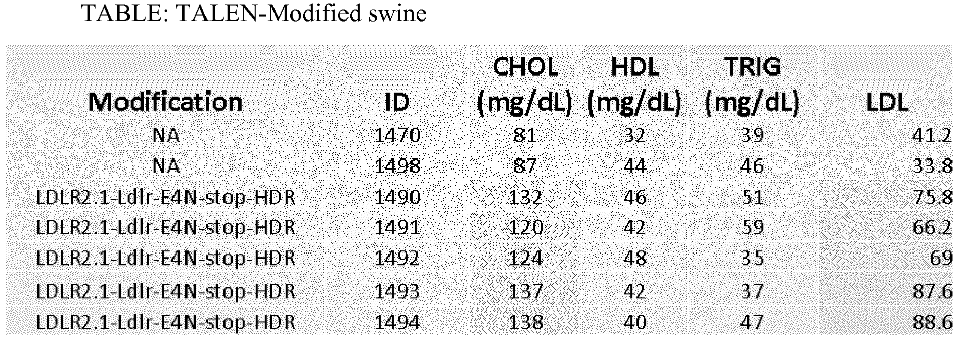

### References

Patent applications, patents, publications, and journal articles set forth herein are hereby incorporated herein by reference for all purposes; in case of conflict, the specification is controlling.
M. CHRISTIAN, et al., Targeting DNA double-strand breaks with TAL effector nucleases, 186 Genetics (2010).
J.C. MILLER, et al., A TALE nuclease architecture for efficient genome editing, 29 Nature Biotech. (2011).
D. A. MCGREW & K. L. KNIGHT, Molecular design and functional organization of the RecA protein, 38 Crit Rev Biochem Mol Biol (2003).
M. M. Cox, Recombinational DNA repair in bacteria and the RecA protein, 63 Prog Nucleic Acid Res Mol Biol (1999).
B. REISS, et al., RecA protein stimulates homologous recombination in plants, 93 Proc Natl Acad Sci U S A (1996).
B. REISS, et al., RecA stimulates sister chromatid exchange and the fidelity of double-strand break repair, but not gene targeting, in plants transformed by Agrobacterium, 97 Proc Natl Acad Sci U S A (2000).
O. G. SHCHERBAKOVA, et al., Overexpression of bacterial RecA protein stimulates homologous recombination in somatic mammalian cells, 459 Mutat Res (2000).
R. J. YANEZ & A. C. PORTER, Gene targeting is enhanced in human cells overexpressing hRAD51, 6 Gene Ther (1999).
Z. CUI, et al., RecA-mediated, targeted mutagenesis in zebrafish, 5 Mar Biotechnol (NY) (2003).
N. TAKAHASHI & I. B. DAWID, Characterization of zebrafish Rad52 and replication protein A for oligonucleotide-mediated mutagenesis, 33 Nucleic Acids Res (2005).
T. CERMAK, et al., Efficient design and assembly of custom TALEN and other TAL effector-based constructs for DNA targeting, (in press) Nucl. Acids Res. (2011). D. F. CARLSON, et al., Strategies for selection marker-free swine transgenesis using the Sleeping Beauty transposon system, 20 Transgenic Res (2011).
A.M. GEURTS, et al., Knockout rats via embryo microinjection of zinc-finger nucleases, 325 Science (2009).
I.D. CARBERY, et al., Targeted genome modification in mice using zinc-finger nucleases, 186 Genetics (2010).
T. MASHIMO, et al., Generation of knockout rats with X-linked severe combined immunodeficiency (X-SCID) using zinc-finger nucleases, 5 PLoS One (2010).
L. TESSON, et al., Knockout rats generated by embryo microinjection of TALENs, 29 Nat Biotechnol (2011).
C. J. PALGRAVE, et al., Species-specific variation in RELA underlies differences in NF-kappaB activity: a potential role in African swine fever pathogenesis, 85 J Virol (2011).
C. MUSSOLINO, et al., A novel TALE nuclease scaffold enables high genome editing activity in combination with low toxicity, Nucleic Acids Res (2011).
D. Y. GUSCHIN, et al., A rapid and general assay for monitoring endogenous gene modification, 649 Methods Mol Biol (2010).
Y. DOYON, et al., Transient cold shock enhances zinc-finger nuclease-mediated gene disruption, 7 Nat Methods (2010).
H.J. KIM, et al., Targeted genome editing in human cells with zinc finger nucleases constructed via modular assembly, 19 Genome Res. (2009).
H. J. LEE, et al., Targeted chromosomal deletions in human cells using zinc finger nucleases, 20 Genome Res (2010).
E. E. PEREZ, et al., Establishment of HIV-1 resistance in CD4+ T cells by genome editing using zinc-finger nucleases, 26 Nat Biotechnol (2008).
D. J. BLAKE, et al., Function and genetics of dystrophin and dystrophin-related proteins in muscle, 82 Physiol Rev (2002).
L. GROBET, et al., A deletion in the bovine myostatin gene causes the double-muscled phenotype in cattle, 17 Nat Genet (1997).
R. KAMBADUR, et al., Mutations in myostatin (GDF8) in double-muscled Belgian Blue and Piedmontese cattle, 7 Genome Res (1997).

### Preferred Embodiments

(1) A method of creating a genetic modification comprising exposing a primary cell in an *in vitro* culture or an embryo to a nucleic acid encoding a TALEN, wherein the cell is a livestock cell and the embryo is a livestock embryo.
(2) The method of (1) wherein the primary cell is exposed to the nucleic acid in the *in vitro* culture.
(3) The method of (1) wherein the nucleic acid encoding a TALEN comprises an mRNA.
(4) The method of (1) wherein the TALEN is a left TALEN and further comprising a right TALEN that cooperates with the left TALEN to make a double strand cut in a DNA.
(5) The method of (1) wherein a second vector comprises the nucleic acid encoding the TALEN.
(6) The method of (1) further comprising exposing the cell or the embryo to a vector encoding a reporter.
(7) The method of (6) further comprising a vector encoding a selection marker.
(8) The method of (6) wherein the reporter comprises a selection marker.
(9).. The method of (6) further comprising choosing a cell or embryo that expresses the reporter for further processing.
(10) The method of (9) wherein the cell is chosen based on elimination of cells from the culture that do not express the reporter.
(11) The method of (9) wherein the reporter comprises a fluorescent biomolecule.
(12) The method of (9) wherein the embryo is chosen based on expression of the reporter.
(13) The method of (9) wherein the cell is chosen based on expression of the reporter.
(14) The method of (6) wherein the vector comprises a plasmid.
(15) The method of (6) wherein the vector comprises a transposon that encodes the reporter, and further comprising a vector that encodes a transposase that recognizes the transposon.
(16) The method of (15) wherein the transposase is chosen from the group consisting of Sleeping Beauty, Frog Prince, Tol2 , Minos , Hsmar, Plaice, and Passport.
(17) The method of (1) further comprising choosing a cell or embryo that expresses the reporter and using the cell or embryo to create a genetically modified animal, with the genetic modification being at a DNA site specifically bound by the TALEN.
(18) The method of (17) further comprising breeding the animal and selecting progeny that express the genetic modification and are free of exogenous reporters.
(19) The method of (18) wherein the animals are homozygous bi-allelic for the modification.
(20) The method of (1) further comprising cloning a genetically modified animal from the cell of the culture wherein the cell comprises a genetic modification at a DNA site specifically bound by the TALEN.
(21) The method of (20) wherein the cell is used to clone the animal by somatic cell nuclear transfer or chromatin transfer.
(22) The method of (1) wherein the genetic modification is chosen from the group consisting of an insertion, a deletion, the insertion of an exogenous nucleic acid fragment, an inversion, a gene conversion to natural allele, gene conversion to a synthetic allele, and a gene conversion to a novel allele.
(23) The method of (1) further comprising delivering a recombinase to the cell or embryo.
(24) The method of (23) wherein the recombinase is delivered as a protein, an mRNA, or by a vector that encodes the recombinase.
(25) The method of (23) wherein the recombinase combines with a nucleic acid to form a filament, with the nucleic acid providing a template for homology dependent repair.
(26) The method of (23) wherein the recombinase is chosen from the group consisting of RecA, recA803, uvsX, recA mutants, recA-like recombinases, Cre recombinase, Hin recombinase, RAD51, Tre, FLP, RuvC, DST2, KEM1 XRN1, STPa/DST1, and HPP-1.
(27) The method of (1) wherein the cell is chosen from the group consisting of a livestock cell, an artiodactyl cell, a cultured cell, a primary cell, a primary somatic cell, a zygote, a primordial germ cell, or a stem cell and a zygote, or wherein the embryo is a blastocyst.
(28) A cell or an embryo comprising a nucleic acid fragment encoding a TALEN and a genetic modification at a DNA site that is specifically bound by the TALEN, wherein the cell comprises a primary cell isolated from an animal tissue, and wherein the cell and the embryo are chosen from the group consisting of artiodactyls, swine, bovine, fish, rabbit, and livestock.
(29) The cell or embryo of (28) comprising the cell, wherein the primary cell is exposed to the nucleic acid in an *in vitro* culture.
(30) The cell or embryo of (28) wherein the nucleic acid encoding a TALEN comprises an mRNA.
(31) The cell or embryo of (28) further comprising a vector encoding a reporter.
(32) The cell or embryo of (28) further comprising a vector encoding a selection marker.
(33) The cell or embryo of (31) wherein the reporter comprises a selection marker.
(34) The cell or embryo of (28) further comprising a vector encoding a reporter, and a vector that encodes the TALEN.
(35) The cell or embryo of (34) further comprising a vector encoding a transposase, with the reporter and the TALEN being transposons encoded by their respective vectors.
(36) The cell or embryo of (28) comprising the embryo, wherein the embryo is homozygous bi-allelic for the genetic modification.
(37) The cell or embryo of (28) further comprising an exogenous recombinase.
(38) A genetically modified livestock animal comprising a genetic modification chosen from the group consisting of an insertion, a deletion, the insertion of an exogenous nucleic acid fragment, an inversion, a gene conversion to natural allele, a gene conversion to a synthetic allele, and a gene conversion to a novel allele, with the animal being free of exogenous reporter genes.
(39) The livestock animal of (38) being chosen from the group consisting of cows, swine, sheep, goats, rabbits, poultry, and fish.
(40) The livestock animal of (38) wherein the genetic modification comprises a deletion that comprises deletion of 500 basepairs.
(41) The livestock animal of (38) wherein the genetic modification comprises a deletion that comprises deletion of a megabasepair.
(42) The livestock animal of (38) wherein the genetic modification comprises an inversion that comprises a megabase of chromosomal DNA.
(43) The livestock animal of (38) wherein the genetic modification comprises an inversion that comprises 500 basepairs.
(44) The livestock animal of (38) wherein the genetic modification comprises an inversion, with the inversion encoding a genetic trait.
(45) The livestock animal of (38) comprising a bovine line of animals that are not *Belgian Blue* cattle, wherein the genetic modification comprises a myostatin deletion prevalent in *Belgian Blue* cattle.
(46) A method of genetically modifying a livestock animal comprising selecting a trait and inverting a region of DNA that encodes the trait in a cell or embryo that is used as a progenitor for a livestock animal.
(47) The method of (46) wherein the inversion comprises 1000 basepairs.
(48) The method of (46) wherein the inversion comprises a megabase of chromosomal DNA.
(49) A method of genetically modifying an animal comprising exposing a progenitor cell or embryo to a first TALEN that specifically binds at a first DNA site and a second TALEN that specifically binds at a second DNA site, with the region between the site being deleted.
(50) The method of (49) wherein the first TALEN comprises a first TALEN pair and the second TALEN comprises a second TALEN pair.
(51) The method of (49) comprising the progenitor cell, wherein somatic cell nuclear transfer or chromatin transfer from the progenitor cell is used to create the animal.
(52) The method of (49) comprising introducing the TALENs into the progenitor cell and also introducing a vector comprising a reporter, and selecting the progenitor cell or embryo for creation of the animal only if the reporter is expressed.
(53) A method of transfer of an allele from a first livestock breed to a second livestock breed comprising
   introducing a TALEN into a cell or embryo of the second livestock breed in a presence of a nucleic acid that encodes the allele of the first livestock breed wherein the allele is copied into the cell or the embryo, and creating an animal of the second breed from the cell or the embryo.
(54) The method of (53) wherein the allele comprises a myostatin allele present in Belgian Blue cattle.
(55) The method of (53) wherein the TALEN is encoded by an mRNA or by a vector.
(56) The method of (53) wherein the TALEN is a right TALEN and further comprising a left TALEN.
(57) The method of (53) further comprising introduction of a reporter vector independently of the TALEN.
(58) The method of (53) wherein the allele is linked to a trait of the first livestock breed.
(59) The method of (53) further comprising delivering a recombinase to the cell or embryo.
(60) The method of (59) wherein the recombinase is delivered as a protein, an mRNA, or by a vector that encodes the recombinase.
(61) The method of (59) wherein the recombinase combines with the nucleic acid to form a filament.
(62) A genetically modified livestock animal comprising
   a first breed of livestock animal comprising an allele of a second breed of livestock animal,
   wherein the first breed of livestock animal is free of meiotic recombination with the second breed of animal.
(63) The animal of (62) wherein the animal is chosen from the group consisting of swine, cows, sheep, goats, chickens, rabbits, and fish.
(64) The animal of (62) wherein the first breed is Wagyu cattle and the second breed is Belgian Blue cattle.
(65) The animal of (62) wherein the allele is chosen from the group consisting of an insertion, a deletion, a polymorphism, and a single nucleotide polymorphism.
(66) The animal of (62) wherein the animal is free of exogenous marker genes.
(67) The animal of claim (62) comprising a plurality of the alleles.
(68) The animal of (62) wherein the allele is linked to a quantitative trait or qualitative trait of the second breed.

## Claims

1. A method of creating a genetic modification in a livestock animal comprising exposing a primary cell in an *in vitro* culture or an embryo to a nucleic acid encoding a TALEN and a homology dependent repair (HDR) template, wherein the cell is a livestock cell and the embryo is a livestock embryo and an exogenous sequence in the HDR template is copied into a_genome of the cell.

2. The method of claim 1, wherein the primary cell or the embryo are from a line.

3. The method of claim 2 wherein the primary cell is exposed to the nucleic acid in the *in vitro* culture.

4. The method of any of claims 1-3 wherein the nucleic acid encoding a TALEN comprises an mRNA.

5. The method of any of claims 1-4 wherein the TALEN is a left TALEN and further comprising a right TALEN that cooperates with the left TALEN to make a double strand cut in a DNA.

6. The method of any of claims 1-3 or 5 wherein a vector comprises the nucleic acid encoding the TALEN.

7. The method of any of claims 1-5 further comprising exposing the cell or the embryo to a vector encoding a reporter.

8. The method of claim 7 further comprising choosing a cell or embryo that expresses the reporter for further processing to make the animal.

9. The method of claim 6 or 7 wherein the vector comprises a transposon that encodes the reporter.

10. The method of any of claims 1-9 wherein the genomically edited livestock animals-is homozygous bi-allelic for the genetic modification.

11. The method of any of claims 1-10 further comprising cloning a genetically modified livestock animal from the cell of the culture wherein the cell comprises the genetic modification at a DNA site specifically bound by the TALEN.

12. The method of claim 11 wherein the cell is used to clone the animal by somatic cell nuclear transfer or chromatin transfer.

13. The method of any of claims 1-12 wherein the genetic modification is chosen from the group consisting of an insertion, a deletion, the insertion of an exogenous nucleic acid fragment, an inversion, a gene conversion to natural allele, gene conversion to a synthetic allele, and a gene conversion to a novel allele.

14. The method of any of claims 1-13 wherein the cell is chosen from the group consisting of a livestock cell, an artiodactyl cell, a cultured cell, a primary cell, a primary somatic cell, a zygote, a primordial germ cell, or a stem cell and a zygote, or wherein the embryo is a blastocyst.
